# EUROPÄISCHE PATENTSCHRIFT

(11) **EP 2 806 915 B1**
(45) Veröffentlichungstag und Bekanntmachung des Hinweises auf die Patenterteilung: **18.05.2016**
(21) Anmeldenummer: 14715869.5
(22) Anmeldetag: 27.03.2014
(51) Int. Cl.: A61M 1/16, A61M 1/34, A61M 1/36, A61B 5/1455

(54) **SYSTEM ZUR ERFASSUNG EINES ZUSTANDS EINER DIALYSATORVORRICHTUNG, UND HIERFÜR VERWENDBARE SENSORVORRICHTUNG**
SYSTEM FOR DETECTING A STATE OF A DIALYZER APPARATUS, AND SENSOR DEVICE WHICH CAN BE USED FOR THIS PURPOSE
SYSTÈME POUR DÉTECTER UN ÉTAT D'UN DISPOSITIF DE DIALYSE, ET DISPOSITIF DE DÉTECTION UTILISABLE À CET EFFET

(30) Priorität: 03.04.2013 DE 102013103335
(43) Veröffentlichungstag der Anmeldung: 03.12.2014
(73) Patentinhaber: B. Braun Avitum AG, 34212 Melsungen (DE)
(72) Erfinder: STROHHÖFER, Christof, Dr., 34123 Kassel (DE); KRAUSE, Silvie, 34212 Melsungen (DE)
(74) Vertreter: Winter, Brandl, Fürniss, Hübner, Röss, Kaiser, Polte - Partnerschaft mbB
(86) Internationale Anmeldenummer: PCT/EP2014/056212
(87) Internationale Veröffentlichungsnummer: WO 2014/161771

(56) Entgegenhaltungen:
- EP-A1- 0 552 014
- WO-A1-02/34314
- WO-A1-98/17193
- DE-A1- 19 627 595
- US-A1- 2006 283 801
- US-A1- 2007 007 184
- US-A1- 2012 095 351

## Beschreibung

Die Erfindung betrifft unter anderem ein System zur Erfassung eines Zustands, z.B. einer Dialysance, und optional zur Identifizierung einer Dialysatorvorrichtung oder einer Komponente derselben wie etwa einer Membranfiltereinrichtung der Dialysatorvorrichtung vor oder während des Betriebs der Dialysatorvorrichtung, z.B. bei einer Blutbehandlung eines Patienten. Weiterhin betrifft die Erfindung eine Sensorvorrichtung zur Erfassung eines Zustands und optional zur Identifizierung eines Dialysators, im Folgenden auch als Dialysatorvorrichtung bezeichnet, oder einer Komponente hiervon wie etwa einer Membranfiltereinrichtung der Dialysatorvorrichtung; eine Dialysatorvorrichtung, die für das Erfassen oder Messen derartiger Information mittels einer derartigen Sensorvorrichtung ausgebildet ist; ein System, das eine derartige Sensorvorrichtung und eine derartige Dialysatorvorrichtung umfasst; sowie eine Dialysemaschine mit einer derartigen Sensorvorrichtung und einer derartigen Dialysatorvorrichtung.

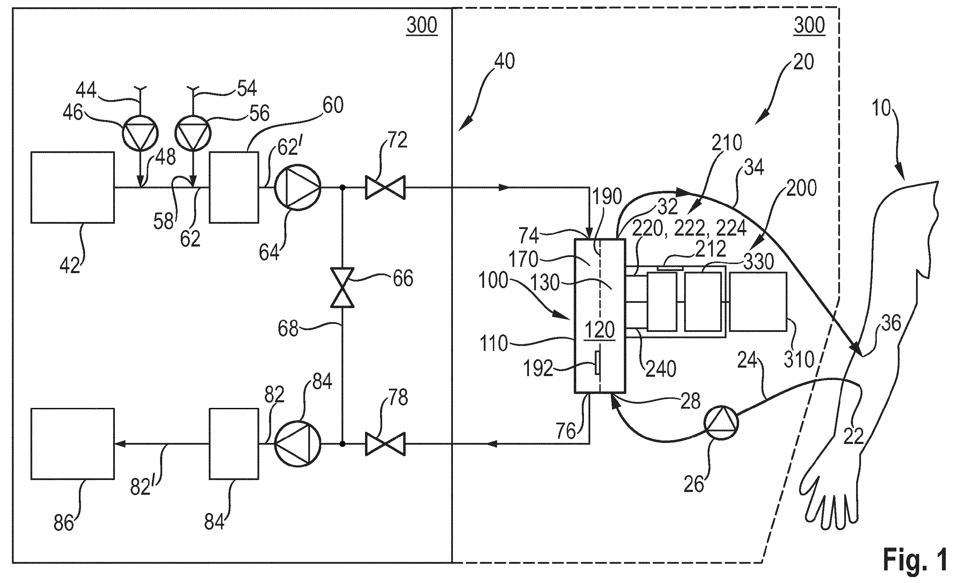

Zur Einordnung der .Erfindung vor dem Hintergrund des Stands der Technik wird zunächst auf die Fig. 1 verwiesen. Fig. 1 zeigt eine Dialysemaschine 300 zum Durchführen einer Hämodialysebehandlung eines Patienten 10 in einem Dialysebetrieb. Die Dialysemaschine 300 kann alternativ oder zusätzlich zur Durchführung einer Hämofiltration oder Hämodiafiltration ausgelegt sein, und umfasst ggf. einen Dialysatströmungspfad oder Dialysatflüssigkeitskreislauf 40, hierin auch Dialysatkreislauf 40 genannt, einen in Bezug auf den Patienten 10 extrakorporal geführten Blutströmungspfad oder Blutkreislauf 20 und eine Dialysatorvorrichtung 100 mit einem Innenvolumenbereich 120 und einer darin angeordneten Membranfiltereinrichtung 190 mit einer Membran. Die Dialysatorvorrichtung 100 bewirkt die Realisierung der Blutbehandlung wie etwa eine Hämodialysebehandlung des Patienten 10, weil in ihr bzw. in der darin angeordneten Membran der Stoffaustausch zwischen dem im Blutkreislauf 20 geführten Blut des Patienten 10 und dem im Dialysatkreislauf 40 geführten Dialysat stattfindet. Mit dem Begriff 'Dialysat' ist hier und im folgenden Text die Dialysierflüssigkeit bzw, die verbrauchte Dialysierflüssigkeit bezeichnet.

Die Membran der Membranfiltereinrichtung 190 trennt den Innenvolumenbereich 120 in den vom Blut des Patienten 10 durchströmten Blutbereich 130 und den vom Dialysat durchströmten Dialysatbereich 170 räumlich voneinander. Der Stoffaustausch durch die Membran umfasst den durch einen Konzentrationsgradienten der im Blut gelösten harnpflichtigen, teilweise toxischen

Stoffe getriebenen Durchtritt derselben durch die Membran in das Dialysat und umgekehrt den durch einen Konzentrationsgradienten der im aufbereiteten Dialysat gelösten Stoffe getriebenen Durchtritt derselben durch die Membran in das Blut des Patienten. Die im aufbereiteten Dialysat enthaltenen Stoffe werden von Komponenten 42, 44, 54 der Dialysataufbereitung in einer für den Patienten 10 spezifisch aufbereiteten Konzentrationsverteilung in das Dialysat eingebracht und gelöst.

Es sind Einrichtungen bekannt, mit deren Hilfe außerhalb der Dialysatorvorrichtung 100 Messungen von Parametern vorgenommen werden können, aus welchen Parametern auf die Leistungsfähigkeit, z.B. die Dialysance der Dialysatorvorrichtung bzw. die Clearance des extrakorporalen Blutkreislaufs geschlossen werden kann. Ebenfalls sind entsprechende Verfahren zum Auswerten der von den Einrichtungen gemessenen Parameter und zum Bereitstellen von Information über die Leistungsfähigkeit einer Dialysatorvorrichtung bekannt.

WO 98/17193 A1 offenbart ein System und ein Verfahren für nichtinvasive hämodynamische Messungen in Hämodialyseshunts. Eine Emitter/Detektorvorrichtung ermöglicht eine Erfassung eines durch einen Photoemitter zu einer Küvette gerichteten Strahlungsanteils durch einen Photodetektor. Dabei ist eine Küvette an jedem Ende und konkret vor einem Eingangsport bzw. nach einem Ausgangsport eines Dialysators angeordnet, und ist an jeder Küvette jeweils ein Photoemitter und ein Photodetektor angebracht. Der Photoemitter und der Photodetektor werden durch eine Federwirkung entfaltende C-Klemme zusammengehalten.

Die Erfindung beruht unter anderem auf der Erkenntnis, dass eine berührungslose Messung von Informationen bzw. Parametern bezüglich der durch einen Dialysator hindurchströmenden Flüssigkeiten (Dialysat bzw. Blut) und/oder eine Messungen bezüglich einer Identifizierung, beispielsweise eines Typs, einer Dialysatorvorrichtung bzw. einer in der Dialysatorvorrichtung angeordneten Membranfiltereinrichtung vorteilhaft ist. Mit Ausführungsbeispielen der Erfindung kann direkt am Dialysator gemessen oder eine Identifizierung durchgeführt werden. Dies ist vorteilhaft, weil der Dialysator aus therapeutischer Sicht den kritischsten Teil einer Dialysemaschine darstellt, weil dort der Stoffaustausch stattfindet und dort die Dialyseeffizienz wesentlich beeinflusst wird. Auch steht im Dialysator aufgrund der Geometrie das Blut in starker Wechselwirkung mit der Membran der Membranfiltereinrichtung, so dass die stärksten Effekte auf blut- bzw. dialysatseitige Parameter gerade im Dialysator stattfinden. Hierbei kann auch berücksichtigt werden, dass sich für die Dialyseeffizienz indikative Parameter während der Verweilzeit des Bluts im Dialysator verändern können, wie etwa Hämatokrit und Koagulationseigenschaften des Bluts, die außerhalb des Dialysators durch Kompensationsmechanismen wieder in ihren Ursprungszustand zurückkehren. Beispielsweise nimmt der Hämatokrit aufgrund der Druckverhältnisse in modernen, auf hohen Durchfluss hin optimierten Dialysatoren über die Länge des Dialysators, insbesondere über die Länge des Blutbereichs, zunächst zu und dann wieder ab. Dieser Verlauf der Hämatokritkonzentration im Dialysator kann bisher nicht direkt gemessen werden, obwohl bekannt ist, dass die maximale Hämatokritkonzentration ein entscheidender Parameter für Koagulations-, Verclottungs- und Hämolyseeffekte ist. Auch enthalten lokale Konzentrationen und Konzentrationsunterschiede der im Dialysat gelösten Stoffe wichtige Informationen über die Qualität der Blutreinigung im Dialysator entlang der Länge der Membran. Eine bei Ausführungsbeispielen vorgesehene Messung bzw. Überwachung entsprechender Parameter, wie z.B. beispielsweise des Koagulationszustands, direkt am Dialysator ist daher vorteilhaft.

Mit der Erfindung wird die Aufgabe gelöst, eine wieder verwendbare Sensorvorrichtung bzw. ein System mit einer wieder verwendbaren Sensorvorrichtung zu schaffen, die bzw. das es ermöglicht, möglichst direkte Informationen bezüglich des Zustands und/oder einer Identifizierung einer als Einmalartikel ausgebildeten Dialysatorvorrichtung oder einer als Einmalartikel ausgebildeten Membranfiltereinrichtung der Dialysatorvorrichtung direkt an der Dialysatorvorrichtung zu messen.

Gemäß einer oder mehreren Ausführungsformen wird eine Vorrichtung zum Messen von Parametern bereitgestellt, aus denen Information bezüglich einer Identifizierung einer Dialysatorvorrichtung oder einer Membranfiltereinrichtung der Dialysatorvorrichtung während ihres Betriebs in einer Dialysebehandlung eines Patienten gewonnen werden können. Dabei werden die Messungen direkt an der Dialysatorvorrichtung vorgenommen. Die Dialysatorvorrichtung bzw. zumindest die darin eingesetzte Membranfiltereinrichtung ist ein Einmalprodukt, das in der Regel nur während einer einzigen Dialysebehandlung an einem Patienten genutzt wird und danach entsorgt wird. Daher ist die Wiederverwendbarkeit der Sensoreinrichtung für mehrere Dialysatorvorrichtungen vorteilhaft.

Mit der Erfindung werden ein System mit einer Dialysatorvorrichtung und einer Sensorvorrichtung mit den Merkmalen des unabhängigen Patentanspruchs 1, eine Sensorvorrichtung mit den Merkmalen des Patentanspruchs 12, eine Dialysatorvorrichtung mit den Merkmalen des Patentanspruchs 15 und eine Dialysemaschine mit den Merkmalen des Patentanspruchs 17 geschaffen. Vorteilhafte Weiterbildungen sind Gegenstand der abhängigen Patentansprüche.

Gemäß einem ersten Aspekt der Erfindung werden eine Sensorvorrichtung sowie ein System zum Erfassen oder Messen von Informationen zur Identifizierung einer Dialysatorvorrichtung oder einer Membranfiltereinrichtung der Dialysatorvorrichtung vor und/oder während des Betriebs der Dialysatorvorrichtung in einer Dialysebehandlung eines Patienten bereitgestellt. Das System umfasst bei einem oder mehreren Ausführungsbeispielen eine Dialysatorvorrichtung mit einem einen Innenvolumenbereich umschließenden Gehäuse und einer im Wesentlichen in dem Innenvolumenbereich angeordneten Membranfiltereinrichtung, und eine Sensorvorrichtung, die mit dem Gehäuse der Dialysatorvorrichtung lösbar operativ verbindbar ist und die eine Signalempfangseinrichtung umfasst, die dazu ausgebildet ist, mindestens ein Signal, wie etwa ein Strahlungssignal , z.B. von der Gehäuseoberfläche, von einem an oder in dem Gehäuse angebrachten oder ausgebildeten Identifikationsträger wie etwa einem Etikett, oder aus dem Innenvolumenbereich des Gehäuses zu empfangen, wobei das Signal charakteristisch für den Zustand bzw. die Identifizierung der Dialysatorvorrichtung bzw. der Membranfiltereinrichtung der Dialysatorvorrichtung ist. Das Gehäuse kann optional für ein Signal, wie etwa ein Strahlungssignal, zumindest bereichsweise durchlässig sein, um z.B. Materialien, Substanzen oder Markierungen im Inneren des Gehäuses erkennen zu können. Eine Identifizierung des Dialysators oder von Komponenten desselben kann alternativ oder zusätzlich mittels außen am Gehäuse angebrachter Merkmale bewerkstelligt werden.

Gemäß einem zweiten Aspekt der Erfindung wird eine Sensorvorrichtung bereitgestellt, die mit einem Gehäuse einer Dialysatorvorrichtung lösbar operativ verbindbar ausgebildet ist, wobei in einem mit der Dialysatorvorrichtung verbundenen Zustand ein System gemäß dem ersten Aspekt der Erfindung ausbildbar ist.

Gemäß einem dritten Aspekt der Erfindung wird eine Dialysatorvorrichtung, also vorzugsweise ein Dialysator, mit einem einen Innenvolumenbereich umschließenden Gehäuse und einer im Wesentlichen in dem Innenvolumenbereich angeordneten Membranfiltereinrichtung bereitgestellt, wobei das Gehäuse für ein Signal, wie etwa ein Strahlungssignal, zumindest bereichsweise durchlässig ist. Die Dialysatorvorrichtung kann hierbei so ausgebildet sein, dass mit ihr eine Sensorvorrichtung gemäß dem zweiten Aspekt der Erfindung lösbar operativ verbindbar und in einem verbundenen Zustand ein System gemäß dem ersten Aspekt der Erfindung ausbildbar ist.

Gemäß einem vierten Aspekt der Erfindung wird eine Dialysemaschine zum Durchführen einer Dialysebehandlung an einem Patienten bereitgestellt. Die Dialysemaschine umfasst eine Dialysatorvorrichtung gemäß dem dritten Aspekt der Erfindung und eine Sensorvorrichtung gemäß dem zweiten Aspekt der Erfindung, wodurch ein System gemäß dem ersten Aspekt der Erfindung ausgebildet werden kann. Die Sensorvorrichtung kann mit diesem derartigen System lösbar operativ verbunden werden.

Die mit der Dialysatorvorrichtung lösbare und operativ verbindbare Ausgestaltung der Sensorvorrichtung ermöglicht, dass die Sensorvorrichtung wiederholt bzw. an mehreren Dialysatorvorrichtungen zum Messen der Information verwendet werden kann, während die Dialysatorvorrichtung oder Teile davon als Einmalartikel ausgebildet sein können.

Die direkte Ankoppelbarkeit der Sensorvorrichtung an der Dialysatorvorrichtung, d.h. also am Dialysator, ermöglicht, dass die Messung von Informationen direkt an der Dialysatorvorrichtung durchgeführt werden können und so die gewünschte Information möglichst direkt bzw. detailliert gemessen werden kann.

Die erfindungsgemäße Sensorvorrichtung ermöglicht, eine Identifizierung der Dialysatorvorrichtung oder der Membranfiltereinrichtung der Dialysatorvorrichtung direkt, fehlerfrei und zuverlässig zu erkennen bzw. Informationen bezüglich des Zustands der Dialysatorvorrichtung, insbesondere des Zustands der Membranfiltereinrichtung präzise, detailliert und direkt an der Dialysatorvorrichtung bzw. der Membranfiltereinrichtung zu messen. Derartige Informationen können bezüglich der Effizienz des Stoffaustauschs durch die Membran der Membranfiltereinrichtung indikative Parameter, einschließlich insbesondere der Dialysance umfassen. Die Stoffaustauscheffizienz ist abhängig von der stofflichen Zusammensetzung des aufbereiteten Dialysats, von der stofflichen Zusammensetzung, insbesondere der Konzentrationen von im Blut des Patienten enthaltenen und durch die Dialysebehandlung dem Blut bzw. dem Patienten zu entziehenden Stoffen, und von Vorgängen in der Dialysatorvorrichtung insbesondere auf kapillarem Niveau bzw. in den Poren der Membran der Membranfiltereinrichtung, wie etwa eine z.B. lokalisierte Verstopfung der Membran durch z.B. Filterverclottung. Das zu messende Signal kann so ausgewählt werden, dass es für mindestens eine dieser Größen indikativ ist. Hierbei besteht die Möglichkeit einer ortsaufgelösten Messung von Parametern im Dialysator. Die Messung kann hierbei gleichzeitig oder sequentiell an zwei oder mehr Stellen am oder im Dialysator erfolgen, z.B. entlang des Dialysators in dessen Achsrichtung, entlang seines Umfangs oder auch schräg hierzu z.B. entlang einer oder mehrerer Linien oder an verteilten Orten in sonstiger beliebig verteilter Weise, oder auch an mehreren, zumindest zum Teil in der Radialrichtung des Dialysators aufeinanderfolgend angeordneten Messstellen.

Das System gemäß dem ersten Aspekt der Erfindung kann dazu ausgebildet sein, das gemessene Signal einer Auswerteeinrichtung bereitzustellen, die auf der Grundlage des gemessenen Signals die Dialysatorvorrichtung identifiziert oder zumindest identifizieren kann und ggf. zusätzlich Daten erzeugt, die zum Steuern von Betriebsparametern einer Dialysemaschine gemäß dem vierten Aspekt der Erfindung verwendet werden können.

Die Signalempfangseinrichtung kann dazu ausgebildet sein, Signale einer Strahlung zu detektieren, die beliebiger Art sein kann oder auch aus einer Gruppe ausgewählt sein kann, die z.B. folgendes umfasst:
a) elektromagnetische Strahlung mit einer beliebigen Wellenlänge, z.B. im optischen Bereich wie etwa im Bereich fernen Infrarot (FIR), infraroten (IR), nahinfraroten, sichtbaren und ultravioletten (UV) Lichts,
b) elektromagnetische Strahlung mit einer Wellenlänge oder einem Wellenlängenbereich bzw. der entsprechenden Frequenz oder dem entsprechenden Frequenzbereich aus dem gesamten elektromagnetischen Spektrum wie etwa z.B. im Mikrowellenbereich, im Terahertzbereich, d.h. im Submillimeterstrahlungsbereich, oder im Bereich von Radiowellen, wie etwa in der RFID-Technologie verwendeten Radiowellen, und
c) Ultraschall-Strahlung. Die vielseitige Ausgestaltbarkeit der Signalempfangseinrichtung ermöglicht, dass unterschiedliche Informationen bzw. Arten von Informationen bzw. verschiedenartige Parameter der Dialysatorvorrichtung oder der Membranfiltereinrichtung gemessen werden können.

Die Signalempfangseinrichtung kann einen Empfänger für ein magnetisches, elektrisches oder elektromagnetisches Signal umfassen, wobei das Signal indikativ für eine zu messende Kapazität und/oder eine zu messende Induktivität ist, die charakteristisch für den Zustand bzw. die Identifizierung der Dialysatorvorrichtung und/oder der Membranfiltereinrichtung ist. Ein solches Signal kann z.B. störungsfrei und zuverlässig beispielsweise vom Außenbereich oder auch aus dem Rand- oder Innenvolumenbereich der Dialysatorvorrichtung von dem z.B. an oder außerhalb der Dialysatorvorrichtung befindlichen Empfänger erfasst werden.

Jede der vorstehend beschriebenen Ausführungen der Signalempfangseinrichtung und der damit durchführbaren Messungen von Informationen kann als eine gesonderte Erfindung eingestuft werden, auf die ggf. eine Teilanmeldung eingereicht werden kann.

Die Sensorvorrichtung gemäß dem zweiten Aspekt der Erfindung kann eine Strahlungsaussendeeinrichtung umfassen, die dazu ausgebildet ist, eine vorbestimmte Strahlung in den Innenvolumenbereich des Gehäuses der Dialysatorvorrichtung auszusenden. Eine solche Strahlung kann beispielsweise Strahlung aus dem gesamten elektromagnetischen Spektrum wie etwa Strahlung mit einer Wellenlänge oder einem Wellenlängenbereich bzw. der entsprechenden Frequenz oder dem entsprechenden Frequenzbereich aus dem gesamten elektromagnetischen Spektrum wie etwa z.B. im Mikrowellenbereich, im Terahertzbereich, d.h. im Submillimeterstrahlungsbereich, oder im Bereich von Radiowellen, wie etwa in der RFID-Technologie verwendeten Radiowellen, oder optische Strahlung, oder Ultraschall-Strahlung sein. Entsprechend kann die Signalempfangseinrichtung dazu ausgebildet sein, einen oder mehrere Parameter wie etwa die Intensität, Phase und/oder das Zeitverhalten von Strahlung, die von der Strahlungsaussendeeinrichtung ausgesendet worden ist, zu messen. Dabei kann im Betrieb die gemessene Strahlung das Ergebnis einer für den Zustand der Dialysatorvorrichtung charakteristischen Wechselwirkung sein, die im Innenvolumenbereich des Gehäuses zwischen der Strahlung und einem oder mehreren der folgenden stattgefunden hat: dem Dialysat bzw. dem Blut, einem in dem Dialysat und/oder in dem Blut enthaltenen Stoff, der Membranfiltereinrichtung und einem in bzw. an der Membranfiltereinrichtung festgehaltenen Stoff, der aus dem Blut bzw. dem Dialysat entstammt. Dabei kann zumindest ein Teil der gemessenen Strahlung aus dem Innenvolumenbereich zu der Signalempfangseinrichtung gelangen. Insbesondere kann dabei die Wechselwirkung durch von der Strahlungsaussendeeinrichtung in den Innenvolumenbereich des Gehäuses ausgesendete Strahlung hergerufen worden sein. Das Messen eines Ergebnisses einer elektromagnetischen, z.B. optischen, Wechselwirkung von z.B. in den Innenvolumenbereich eingestrahlter Strahlung ermöglicht berührungslose Messungen an den genannten, z.B. im Innenvolumenbereich im Betrieb vorhandenen Elementen und damit Messungen von direkt den Zustand der Dialysatorvorrichtung charakterisierenden Parametern. Bei der Messung von Strahlung, die zum Beispiel von der Oberfläche oder aus dem Innenbereich des Dialysators stammt und als Reflexion, Streuung, Beugung oder als Frequenzumsetzung von diffuser oder gezielt ein- oder aufgestrahlter elektromagnetischer Strahlung wie etwa Licht erhalten wird, kann bei einem, mehreren oder allen Ausführungsbeispielen eine ortsaufgelöste Messung durchgeführt werden. Ortsaufgelöste Messungen ermöglichen beispielsweise die ortsaufgelöste Erfassung dialysatorspezifischer Eigenschaften. Der Einsatz der Ortsauflösung als Messprinzip erlaubt es z.B., die Beeinflussung wie etwa Beugung oder Ablenkung eines einfallenden Lichtstrahls zu bestimmen und daraus Parameter abzuleiten. Zur Ortsauflösung kann z.B. an einer oder mehreren Positionen gemessen werden, so dass dialysatorspezifische Eigenschaften ortsaufgelöst gemessen werden können. Hierbei kann die Mehrzahl von Messungen zur Erhöhung der Messgenauigkeit miteinander verrechnet werden. Dazu kann die erfasste Strahlung an mehreren Punkten in Richtung, oder quer, oder schräg zu der Strahlungsaus- oder -eintrittsrichtung durch einen oder mehrere Sensoren detektiert werden. Damit ergibt sich eine Ortsauflösung als Messprinzip, z.B. um die Ablenkung einer einfallenden elektromagnetischen Strahlung wie etwa von Licht zu bestimmen und daraus Parameter, z.B. über den Brechungsindex, den Innenaufbau, das Material des Dialysators oder dgl. abzuleiten.

Es kann eine elektromagnetische, z.B. optische, Wechselwirkung hervorgerufen werden, die aus einer Gruppe ausgewählt ist, die folgendes umfasst:
- Reflektion von von der Strahlungsaussendeeinrichtung (240) ausgesendeter optischer Strahlung an einer Grenzfläche zwischen der Gehäusewand (112) und dem Dialysat oder dem Blut,
- Reflektion von von der Strahlungsaussendeeinrichtung (240) ausgesendeter optischer Strahlung an einer Grenzfläche zwischen der Membranfiltereinrichtung (190) und dem Dialysat oder dem Blut,
- Transmission von von der Strahlungsaussendeeinrichtung (240) ausgesendeter optischer Strahlung mit einer Messwellenlänge, die von einem in dem Dialysat und/oder in dem Blut enthaltenen Stoff absorbiert werden kann und die auf ihrem Weg zu der Strahlungsempfangseinrichtung (220) einen Strahlungslaufweg durch das Dialysat und/oder durch das Blut zurückgelegt hat,
- Emission von Lumineszenz- oder Fluoreszenzstrahlung durch einen in dem Dialysat und/oder in dem Blut enthaltenen Stoff, wobei eine Lumineszenz- oder Fluoreszenzreaktion in diesem Stoff durch von der Strahlungsaussendeeinrichtung (240) ausgesendete optischer Strahlung hervorgerufen worden ist,
- Brechung von von der Strahlungsaussendeeinrichtung (240) ausgesendeter optischer Strahlung an einer Grenzfläche zwischen der Gehäusewand (112) und dem Dialysat oder dem Blut, wobei die ausgesendete Strahlung unter einem zwischen 0° und 180° oder 0° und 90° liegenden Einfallswinkel auf der Grenzfläche auftrifft,
- Streuung von von der Strahlungsaussendeeinrichtung (240) ausgesendeter optischer Strahlung an einem in dem Dialysat oder in dem Blut enthaltenen Stoff, einschließlich dynamischer Streuung von monochromatischer Laserstrahlung, und
- Wechselwirkung von von der Strahlungsaussendeeinrichtung (240) ausgesendeter optischer Strahlung mit einer Identifizierungseinrichtung (192), die an der Dialysatorvorrichtung (100) oder an der Membranfiltereinrichtung (190) angebracht ist, die ein für die Identifizierung der Dialysatorvorrichtung (100) oder der Membranfiltereinrichtung (190) charakteristisches Identifizierungsmerkmal aufweist. Hierbei können alle möglichen Codes wie etwa optische, akustische, elektrische, magnetische und sonstige Codes, auch eine mechanische Form der Codierung, z.B. eine spezielle Gehäuseform, welche gleichzeitig ein optisches Element ist, oder eine Texterkennung zum Einsatz kommen.

Die vielseitige Auswahlmöglichkeit der optischen Wechselwirkung, die die gemessene Strahlung im Innenvolumenbereich mit den dort vorhandenen Elementen untergeht, ermöglicht, dass unterschiedliche Informationen bzw. Arten von Informationen bzw. verschiedenartige Parameter zur Identifizierung des Dialysators, oder zur Erfassung der im Innenvolumenbereich vorhandenen Elemente oder der Membranfiltereinrichtung gemessen werden können.

Die Dialysatorvorrichtung oder die Membranfiltereinrichtung können bei einem, mehreren oder allen Ausführungsbeispielen eine sogenannte passive Identifizierungseinrichtung umfassen, die ein für die Identifizierung der Dialysatorvorrichtung oder der Membranfiltereinrichtung charakteristisches Identifizierungsmerkmal aufweist, das z.B. durch Anstrahlen der Identifizierungseinrichtung mit einer vorbestimmten elektromagnetischen Strahlung auslesbar ist. Dabei kann die Sensorvorrichtung eine Strahlungsaussendeeinrichtung umfassen, die dazu ausgebildet ist, die vorbestimmte elektromagnetische Strahlung, insbesondere zu der Identifizierungseinrichtung, auszusenden. Dabei kann die Signalempfangseinrichtung eine Identifizierungsleseeinrichtung umfassen, die dazu ausgebildet ist, einen für das Identifizierungsmerkmal indikativen Parameter der Strahlung zu detektieren und das Identifizierungsmerkmal zu bestimmen. Die Identifizierungseinrichtung kann etwa ein Barcode, ein Farbencodefeld oder ein passiver RFID Chip sein. Die Identifizierungseinrichtung ermöglicht, dass Information bezüglich der Identifizierung der Dialysatorvorrichtung oder der Membranfiltereinrichtung besonders zuverlässig, direkt und im Wesentlichen fehler- und störungsfrei gemessen werden kann.

Alternativ dazu kann die Dialysatorvorrichtung oder die Membranfiltereinrichtung eine sogenannte aktive Identifizierungseinrichtung umfassen, die elektromagnetische Strahlung aussenden kann, die ein für die Identifizierung der Dialysatorvorrichtung oder der Membranfiltereinrichtung charakteristisches Identifizierungsmerkmal aufweist. Dabei kann die Signalempfangseinrichtung eine Identifizierungsleseeinrichtung umfassen, die dazu ausgebildet ist, die von der Identifizierungseinrichtung ausgesendete elektromagnetische Strahlung zu detektieren und, etwa nach einer Demodulationsauswertung, das Identifizierungsmerkmal zu bestimmen. Dabei kann die Identifizierungseinrichtung etwa ein aktiver RFID Chip sein. Mit einer aktiven Identifizierungseinrichtung kann Information bezüglich der Identifizierung der Dialysatorvorrichtung oder der Membranfiltereinrichtung besonders zuverlässig, direkt und im Wesentlichen fehler- und störungsfrei gemessen werden.

Die Strahlungsaussendeeinrichtung kann mindestens eines oder mehrere der folgenden Merkmale umfassen: eine Lichtquelle, die Licht in einem schmalbandigen Spektralbereich aussendet, wie etwa ein Laser oder eine LED, eine Lichtleitfaser, die aus einer Licht in einem schmalbandigen Spektralbereich aussendenden Lichtquelle ausgesendetes Licht führt und einen Auskopplungsabschnitt aufweist, aus dem das Licht austritt, eine Lichtquelle, die Licht in einem breitbandigen Spektralbereich aussendet, wie etwa eine Halogenlampe, oder eine Lichtleitfaser, die aus einer Licht in einem breitbandigen Spektralbereich aussendenden Lichtquelle ausgesendetes Licht führt und einen Auskopplungsabschnitt aufweist, aus dem das Licht austritt. Diese vielseitige Ausgestaltbarkeit der Strahlungsaussendeeinrichtung ermöglicht, dass verschiedenartige optische Wechselwirkung der in den Innenvolumenbereich ausgesendeten Strahlung mit den dort vorhandenen Elementen induziert und im Ergebnis verschiedenartige Parameter bzw. Informationen bezüglich des Zustands der Dialysatorvorrichtung_ bzw. der Membranfiltereinrichtung gemessen werden können.

Die Strahlungsaussendeeinrichtung kann eine 1-dimensionale Anordnung oder eine 2-dimensionale Anordnung von mehreren Strahlungsaustrittsbereichen umfassen. Die Strahlungsaussendeeinrichtung kann einen oder mehrere Sender oder Emitter und optional auch Lichtleiter aufweisen. Bei einem oder mehreren Ausführungsbeispielen kann das Gehäuse selbst als Lichtleiter dienen. Es kann eine ausgezeichnete, also eine bevorzugte z.B. lineare Ausrichtung der 1-dimensionalen oder der 2-dimensionalen Anordnung im Betrieb und im verbundenen Zustand der Sensorvorrichtung und der Dialysatorvorrichtung im Wesentlichen parallel zu einer Strömungsrichtung des Dialysats in dem Dialysatbereich, oder parallel zu der Strömungsrichtung des Bluts in dem Blutbereich, oder parallel zu einer Längsachse der Dialysatorvorrichtung, oder entlang des Umfangs des Gehäuses, oder quer oder schräg zu diesen Ausrichtungen, oder kombiniert in zwei oder mehreren dieser Ausrichtungen, also zwei- oder dreidimensional verteilt, ausgerichtet sein. Die Ausgestaltung der Strahlungsaussendeeinrichtung mit einer 1- oder 2-dimensionalen Anordnung mehrerer Strahlungsaustrittsbereiche ermöglicht, dass die ausgesendete Strahlung räumlich verteilt und räumlich selektiv verteilt in den Innenvolumenbereich der Dialysatorvorrichtung eingestrahlt werden und infolgedessen z.B. bei einer geeigneten Ausbildung der Strahlungsmesseinrichtung die gewünschte Information räumlich selektiv gemessen werden kann.

Die Sensorvorrichtung kann eine Strahlungsmesseinrichtung, z.B. mit einer Strahlungsbeeinflussungseinrichtung wie etwa einer Messoptikeinrichtung, und einen Strahlungs-, z.B. Lichtdetektor, der eine strahlungs- oder lichtsensitive Detektorfläche aufweist, umfassen. Die Funktionalität der Strahlungsbeeinflussungseinrichtung kann auch durch das Gehäuse selbst bereitgestellt werden. Das Gehäuse kann z.B. als Lichtleiter ausgebildet oder mit einem oder mehreren Lichtleitern ausgestattet sein.

Die Strahlungsbeeinflussungseinrichtung oder Messoptikeinrichtung kann optional eine Fokaltiefe und / oder eine Fokalrichtung aufweisen. Die Strahlungsbeeinflussungseinrichtung kann auch ausgebildet sein, um Strahlung oder Licht der Lichtquelle an einen bestimmten Punkt im Dialysator abzubilden oder dorthin zu fokussieren. Alternativ oder zusätzlich kann die Strahlungsbeeinflussungseinrichtung dazu dienen, Licht aus dem Dialysator auf die Sensorvorrichtung (Detektor) zu fokussieren und nicht nur abzubilden. Dabei kann die Strahlungsbeeinflussungseinrichtung wie etwa die Messoptikeinrichtung dazu ausgebildet sein, einen Raumbereich aus dem Innenvolumenbereich des Gehäuses der Dialysatorvorrichtung auf die Detektorfläche des Lichtdetektors abzubilden, wobei der abgebildete Raumbereich durch die Fokaltiefe und die Fokalrichtung definiert ist. Die Strahlungsbeeinflussungseinrichtung kann , so ausgebildet sein, dass ihre Fokaltiefe und Fokalrichtung so wählbar ist, dass der abgebildete Raumbereich im Bereich des Innenvolumenbereichs wählbar ist. Durch diese Ausbildung der Strahlungsmesseinrichtung kann die gewünschte Information an dem Außen- oder Randbereich oder in manchen Fällen auch aus oder in dem Innenvolumenbereich z.B. zur Identifizierung des Dialysators räumlich selektiv gemessen werden.

Die Sensorvorrichtung kann eine Strahlungsmesseinrichtung mit mindestens einem Strahlungseintrittsbereich und mindestens einem Strahlungs-, z.B. Lichtdetektor umfassen, der dazu ausgebildet ist, Strahlung, wie etwa Licht, die in den mindestens einen Strahlungseintrittsbereich eingetreten ist, zu detektieren und der beispielsweise aus einer Gruppe ausgewählt ist, die folgendes umfassen kann: eine Photodiode, einen Phototransistor, einen CMOS-Lichtdetektor, einen Photomultiplier mit einem oder mehreren linienförmig eindimensional oder flächig zweidimensional verteilt angeordneten Multiplierelementen, eine Avalanche-Photodiode mit einem oder mehreren linienförmig eindimensional oder flächig zweidimensional verteilt angeordneten Photodiodenelementen, einen 1-dimensionalen oder 2-dimensionalen CCD-Sensor, und eine 1-dimensionale Anordnung oder eine 2-dimensionale Anordnung einer Vielzahl von Photodioden, Phototransistoren, CMOS-Lichtdetektoren, Photomultiplier oder Avalanche-Photodioden, oder andere Detektoren wie etwa MPPC (Multi Photon Pixel Counter, oder sonstige Sensorelemente. Die vielseitige Wählbarkeit der Art des Strahlungsdetektors ermöglicht es, für die Messung verschiedenartiger Parameter bzw. Informationen jeweils optimal geeignete Strahlungsdetektoren bereitzustellen.

Ein jeweiliger Strahlungseintrittsbereich kann einen Einkoppelbereich eines optischen Lichtleiters oder einer Lichtleitfaser umfassen, der/die dazu ausgebildet ist, die Strahlung zu einem dem Strahlungseintrittsbereich zugeordneten Lichtdetektor zu leiten. Diese Ausgestaltung ermöglicht es, den Strahlungseintrittsbereich und den diesem zugeordneten Lichtdetektor räumlich voneinander beabstandet einzurichten bzw. räumlich zu entkoppeln. So können ggf. auch Lichtdetektoren zum Einsatz kommen, die räumlich relativ viel Platz einnehmen, um Strahlung zu messen, die dicht an der Dialysatorvorrichtung an dem Strahlungseintrittsbereich "aufgefangen" wird.

Die Sensorvorrichtung kann eine Strahlungsmesseinrichtung mit mindestens einem Lichtdetektor und einer Wellenlängenauswahleinrichtung, wie etwa einem Monochromator, umfassen, wobei die Wellenlängenauswahleinrichtung dazu ausgebildet ist, einen schmalbandigen Wellenlängenbereich, der eine Nachweiswellenlänge umfasst, aus einem beispielsweise breitbandigeren Wellenlängenbereich der von der Strahlungsaussendeeinrichtung ausgesendeten Strahlung, z.B. Licht, so auszuwählen, dass eine optische Wechselwirkung, wie etwa Absorption oder Anregung von Lumineszenz- oder Fluoreszenzemission, mit einem ausgewählten Stoff, der sich im Betrieb in dem Innenvolumenbereich des Gehäuses befindet, erfolgt. Dabei kann der Stoff etwa ein Bestandteil des Dialysats und/oder des Bluts sein. Die in dieser Ausgestaltung ermöglichte wellenselektive Messung von Strahlung, z.B. Licht, ermöglicht auch eine stoffspezifische Messung.

Die Anmelderin behält sich vor, auf die hiervor beschriebenen Ausführungen der Sensorvorrichtung mit den verschiedenartigen Strahlungsaussendeeinrichtungen und Strahlungsmesseinrichtungen und der damit induzierbaren optischen Wechselwirkungen und durchführbaren Messungen von Information bezüglich des Zustands der Dialysatorvorrichtung oder der Membranfiltereinrichtung eine gesonderte Schutzrechts- bzw. Patentanmeldung, insbesondere eine Teilanmeldung einzureichen.

Die Sensorvorrichtung kann für eine Aufgabe ausgebildet sein, die mindestens einer der folgenden umfasst: im Blutbereich des Innenvolumenbereichs, Messen eines für die Konzentration eines urämischen Toxins indikativen Parameters, wie etwa die Absorption von Licht mit einer Messwellenlänge, das von dem urämischen Toxin absorbiert wird; im Dialysatbereich des Innenvolumenbereichs, Messen eines für die Konzentration eines urämischen Toxins indikativen Parameters, wie etwa die Absorption von Licht mit einer Messwellenlänge, das von dem urämischen Toxin absorbiert wird; und im Blutbereich, Messen eines für eine physikalische Eigenschaft des Bluts, wie etwa Viskosität oder Hämatokrit- Konzentration, indikativen Parameters. Die Möglichkeit der Ausbildung der Sensorvorrichtung für die genannten, unterschiedlichen Aufgaben ermöglicht, das in der Praxis häufig verwendete und/oder den Fortgang der Dialysetherapie am Patienten kennzeichnende Parameter gemessen werden können. Entsprechend können auf der Grundlage der Messung dieser Parameter Einstellungen bzw. Betriebsparameter einer Dialysemaschine im Verlauf der Dialysetherapie an den Fortgang der Dialysetherapie angepasst gesteuert werden.

Das Gehäuse der Dialysatorvorrichtung gemäß dem dritten Aspekt der Erfindung kann so ausgebildet sein, dass die Sensorvorrichtung in einer oder mehreren vorbestimmten Positionen relativ zu dem Gehäuse formschlüssig lösbar fixiert werden kann. In einer vorteilhaften Ausgestaltung kann das Gehäuse an seiner Außenseite an einer oder mehreren Positionen erste Koppelbereiche aufweisen, und die Sensorvorrichtung kann einen zweiten Koppelbereich aufweisen, der formschlüssig mit dem ersten Koppelbereich eingreifbar, z.B. andrückbar ausgebildet ist. Das Anbringen der Sensorvorrichtung an einer vorbestimmten Position relativ zu dem Gehäuse ermöglicht es, mit einer Sensorvorrichtung die gewünschte Information an unterschiedlichen Dialysatorvorrichtungen zuverlässig und unter vergleichbaren Messbedingungen und damit auch vergleichbar zu messen

Vorzugsweise ist die Sensorvorrichtung gemäß dem zweiten Aspekt der Erfindung wieder verwendbar ausgebildet. Durch die Wiederverwendbarkeit wird es effizient bzw. kostengünstig, auch relativ aufwändige, komplexe und entsprechend in der Anschaffung teure Sensorik einzusetzen.

Die an dem Gehäuse der Dialysatorvorrichtung gemäß dem dritten Aspekt der Erfindung lösbar, insbesondere formschlüssig lösbar, befestigbare Ausbildung der Sensorvorrichtung gemäß dem zweiten Aspekt der Erfindung kann z.B. durch mindestens einen der folgenden Mechanismen bewirkt werden:
die Sensorvorrichtung (200) umfasst einen Grundkörper und eine Clipeinrichtung mit mindestens einem oder zwei an dem Grundkörper befestigten elastisch-flexiblen Cliparmen oder an dem Grundkörper elastisch-flexibel angelenkten Cliparmen, der oder die dazu ausgebildet sind, das Gehäuse (110) zumindest teilweise zu umgreifen oder mit diesem eine feste lösbare Verbindung einzugehen oder aufgrund ihrer elastisch-flexiblen Ausgestaltung oder Anlenkung das Gehäuse (110) clipartig zu umklammern,
die Sensorvorrichtung umfasst einen Grundkörper und mindestens ein Paar von an dem Grundkörper angelenkten oder mit dem Grundkörper elastisch-flexibel verbundenen Armen bzw. Manschetten, die jeweils einen distalen Endbereich mit einem dort vorgesehenen ersten Glied einer Haken- oder Eingreifeinrichtung aufweisen, wobei die Arme bzw. Manschetten dazu ausgebildet sind, das Gehäuse in einem Winkelbereich von mehr als 180°, vorzugsweise mehr als 270°, und mehr bevorzugt mehr als 320°, umgreifen und eine elastisch spannbare Spanneinrichtung mit zwei gegenüberliegenden Endabschnitten, an denen jeweils eine zum lösbaren Eingreifen mit einem ersten Glied der Haken- oder Eingreifeinrichtung ausgebildetes zweites Glied einer Haken- oder Eingreifeinrichtung vorgesehen ist, wobei in einem elastisch gespannten Zustand der Spanneinrichtung ein jeweiliges zweites Glied am Endabschnitt der Spanneinrichtung mit einem ersten Glied an den Endbereichen der Arme bzw. Manschetten der Sensorvorrichtung eingreifen kann, und
die Sensorvorrichtung ist dazu ausgebildet, beispielsweise in einer Vertiefung, in der Gehäusewand der Dialysatorvorrichtung lösbar. integriert zu werden.

Das Gehäuse der Dialysatorvorrichtung gemäß dem dritten Aspekt der Erfindung kann eine Gehäusewand mit mindestens einem für ein Signal durchlässigen Fensterbereich umfassen. Die Ausbildung mit einem für das Signal besonders durchlässigen Fensterbereich ermöglicht, dass auch an einem Gehäuse mit einem ansonsten für das Signal weniger durchlässigen Material der Gehäusewand, die jedoch beispielsweise aus einem kostengünstigen Material hergestellt sein kann, genaue Messungen der gewünschten Information bzw. Parameter mit der an dem bzw. in der Nähe des Fensterbereichs anzubringenden Sensorvorrichtung durchgeführt werden können.

Die Gehäusewand kann im Wesentlichen zylinderförmig ausgebildet sein. Dies ermöglicht, dass die Sensorvorrichtung leicht in entlang einer Längsrichtung der Gehäusewand verschiedenen Positionen und mit in Bezug auf die Längsachse azimutal unterschiedlichen Blickrichtungen angebracht werden kann.

Die Membranfiltereinrichtung kann in das Gehäuse der Dialysatorvorrichtung herausnehmbar eingebracht werden. Dies ermöglicht, dass nur die Membranfiltereinrichtung als Einmalartikel ausgebildet sein kann, während das Gehäuse der Dialysatorvorrichtung, insbesondere z.B. wenn darin relativ teure Fensterbereiche integriert sind, wieder verwendbar sein kann.

Alternativ kann die Dialysatorvorrichtung zusätzlich noch ein Innengehäuse umfassen, das in dem Gehäuse lösbar fixiert werden kann und das die Membranfiltereinrichtung im Wesentlichen umschließt. Dabei kann das Innengehäuse, insbesondere mit der darin angeordneten Membranfiltereinrichtung, in das Gehäuse der Dialysatorvorrichtung herausnehmbar eingebracht werden. Dies ermöglicht, dass das Innengehäuse mit der Membranfiltereinrichtung als Einmalartikel ausgebildet sein kann, während das Gehäuse der Dialysatorvorrichtung, insbesondere z.B. wenn darin relativ teure Fensterbereiche integriert sind, wieder verwendbar sein kann.

Dabei kann zumindest die Membranfiltereinrichtung als Einmalartikel ausgebildet sein, oder können das Innengehäuse und die Membranfiltereinrichtung als Einmalartikel ausgebildet sein. Bei dieser Ausführung kann bei einem oder mehreren Ausführungsbeispielen die Sensorik auch direkt in das Dialysatorgehäuse eingebracht sein, wobei nur die elektrischen Schnittstellen außen am Dialysator kontaktiert werden.

In den vorgenannten Konfigurationen wird bevorzugt, dass das Gehäuse so ausgebildet ist, dass es leicht und gründlich gereinigt werden kann.

Die Dialysatorvorrichtung kann auch als Ganzes als Einmalartikel ausgebildet sein. In dieser Ausgestaltung ist ein Reinigen der Dialysatorvorrichtung oder von Komponenten derselben nach einer an einem Patienten durchgeführten Dialysetherapie nicht erforderlich.

Die Anmelderin behält sich vor, auf die hiervor beschriebenen Ausführungen der lösbar formschlüssig befestigbaren Ausbildung der Sensorvorrichtung und der verschiedenartigen mechanischen bzw. konstruktiven Ausgestaltungen der Dialysatorvorrichtung und der damit erzielbaren Vorteile eine gesonderte Schutzrechts- bzw. Patentanmeldung, insbesondere eine Teilanmeldung einzureichen.

Der Innenvolumenbereich der Dialysatorvorrichtung kann einen im Betrieb von einem Dialysat durchströmbaren Dialysatbereich und einen im Betrieb von Blut durchströmbaren Blutbereich umfassen, wobei der Dialysatbereich von dem Blutbereich durch die Membranfiltereinrichtung räumlich getrennt ist. Die Membranfiltereinrichtung kann dazu ausgebildet sein, im Betrieb einen durch Konzentrationsgradienten von in dem Blut bzw. in dem Dialysat gelösten Stoffen getriebenen Stoffaustausch zwischen dem Blut und dem Dialysat zu ermöglichen. Dies Konfigurationen ermöglichen eine Verwendung der Dialysatorvorrichtung gemäß dem dritten Aspekt der Erfindung in herkömmlichen Dialysemaschinen.

Die Dialysatorvorrichtung oder eine Dialysemaschine gemäß dem vierten Aspekt der Erfindung kann zumindest einen Gehäusehalter umfassen, der dazu ausgebildet ist, im Betrieb mit der Dialysemaschine zumindest mechanisch verbunden zu sein und die Dialysatorvorrichtung an der Dialysemaschine lösbar zu fixieren. Der Gehäusehalter kann die Dialysatorvorrichtung, ggf. im Betrieb zusammen mit der an der Dialysatorvorrichtung angebrachten Sensorvorrichtung, zuverlässig halten.

In der Dialysemaschine gemäß dem vierten Aspekt der Erfindung kann die Sensorvorrichtung gemäß dem zweiten Aspekt der Erfindung in dem Gehäusehalter integriert sein. Diese Ausgestaltung vereinfacht die Handhabung der Sensorvorrichtung, weil diese nicht in einem zusätzlichen Arbeitsschritt an dem Gehäuse der Dialysatorvorrichtung angebracht zu werden braucht.

Die Erfindung wird im Folgenden anhand von in den beigefügten Figuren gezeigten Ausführungsformen der erfindungsgemäßen Sensorvorrichtung und der erfindungsgemäßen Dialysatorvorrichtung näher beschrieben. Es zeigen:
Figur 1 eine schematische Darstellung eines Blutkreislaufes und eines beispielsweise in einer Dialysemaschine angeordneten Dialysatkreislaufes in einem Dialysebetrieb, wobei an den Blutkreislauf als extrakorporaler Blutkreislauf ein Patient angeschlossen ist;

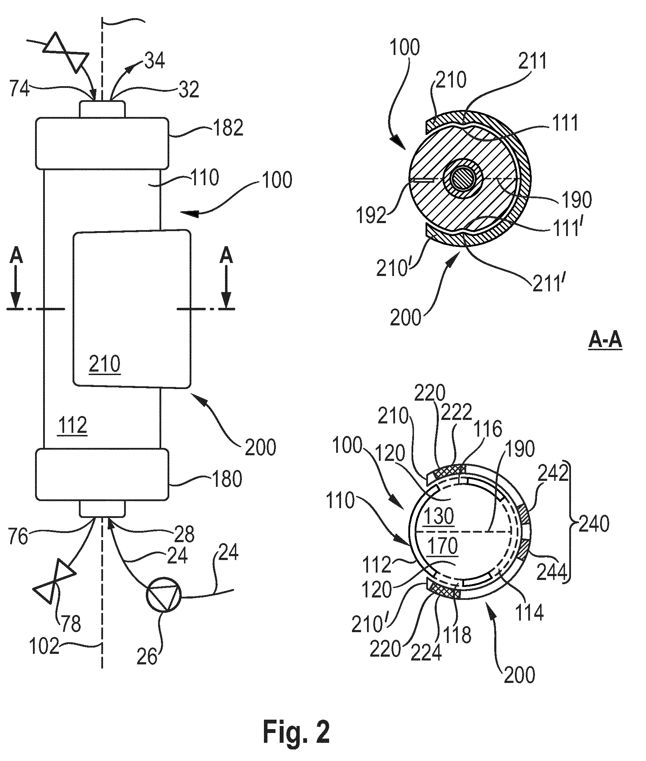

Figur 2 im linken Bereich eine schematische Seitenansicht eines System mit einer ersten Ausführungsform einer Sensorvorrichtung, die an einer ersten Ausführungsform einer Dialysatorvorrichtung lösbar operativ verbunden ist, im mittleren Bereich eine schematische Draufsicht auf das System mit der Sensorvorrichtung und der Dialysatorvorrichtung aus dem linken Bereich, und im rechten Bereich einen schematischen Querschnitt entlang der Linie A-A durch das System mit der Sensorvorrichtung und der Dialysatorvorrichtung aus dem linken Bereich;

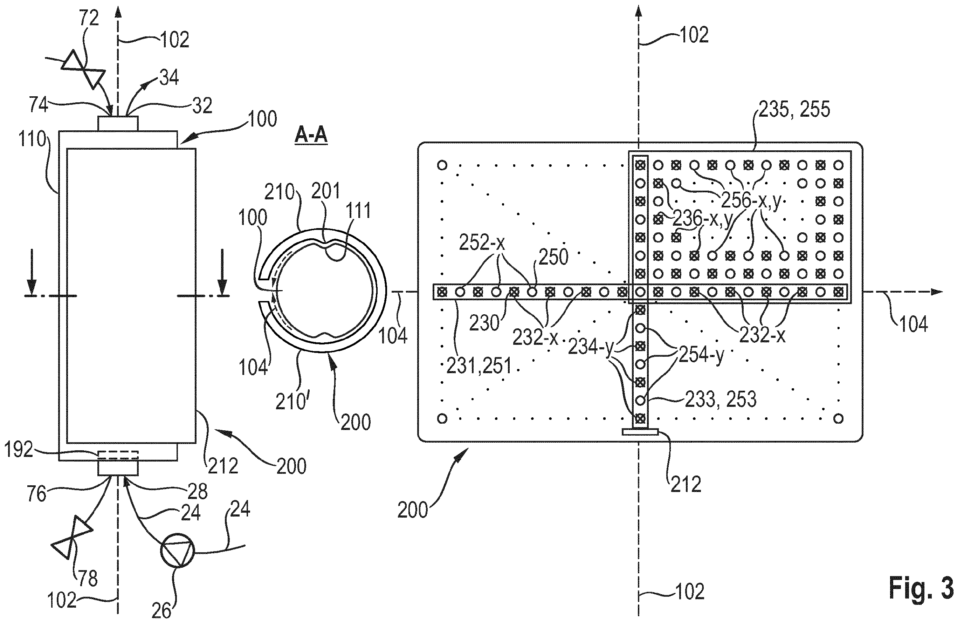

Figur 3 im linken Bereich eine schematische Seitenansicht eines System mit einer zweiten · Ausführungsform einer Sensorvorrichtung, die an einer zweiten Ausführungsform einer Dialysatorvorrichtung lösbar operativ verbunden ist, im mittleren Bereich einen schematischen Querschnitt entlang der Linie A-A durch das System mit der Sensorvorrichtung und der Dialysatorvorrichtung aus dem linken Bereich, und im rechten Bereich eine schematische Darstellung der Sensorvorrichtung aus dem linken Bereich in einem auseinander gerollten Zustand; und

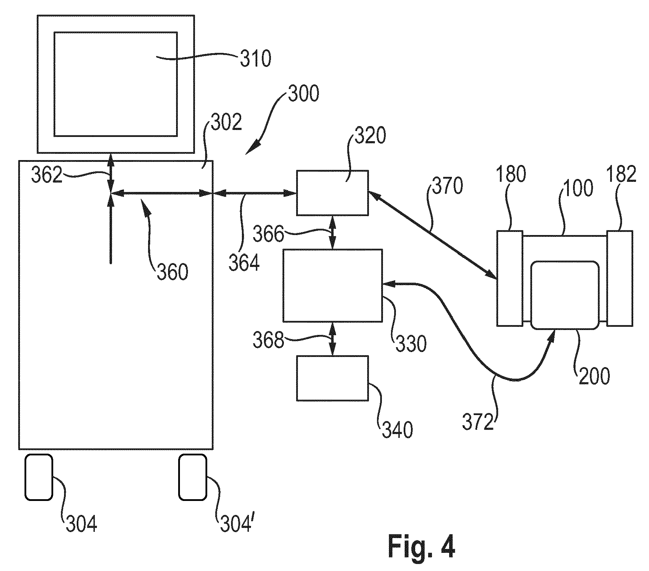

Figur 4 eine Blockdarstellung einer Dialysemaschine und ihrer funktionellen Komponenten einschließlich einer Ausführungsform eines Systems mit einer Ausführungsform einer Sensorvorrichtung, die mit einer Ausführungsform einer Dialysatorvorrichtung lösbar verbunden ist.

Fig. 1 zeigt schematisch eine Dialysemaschine 300 zum Durchführen einer Hämodialysebehandlung eines Patienten 10. Wie eingangs bereits erwähnt, umfasst die Dialysemaschine 300 den Dialysatkreislauf 40, den in Bezug auf den Patienten 10 extrakorporal geführten Blutkreislauf 20 und einen Dialysator, d.h. die Dialysatorvorrichtung 100 mit dem Innenvolumenbereich 120 und der darin angeordneten Membranfiltereinrichtung 190 mit der Membran, an der der Stoffaustausch zwischen dem im Blutkreislauf 20 geführten Blut des Patienten 10 und dem im Dialysatkreislauf 40 geführten Dialysat stattfindet.

Der Dialysatkreislauf 40 kann im Wesentlichen innerhalb der Dialysemaschine 300 angeordnet sein, aber auch anders ausgestaltet sein, und umfasst im Einzelnen die Komponenten 42, 44, 54, 60 zur Dialysataufbereitung, den Dialysatbereich 170 der Dialysatorvorrichtung 100, ein Dialysatoreingangsventil 72 und einen Dialysateinlass 74 zum Einlassen des aufbereiteten Dialysats in den Dialysatbereich 170, einen Dialysatauslass 76 und ein Dialysatorausgangsventil 78 zum Auslassen des gebrauchten Dialysats aus dem Dialysatbereich 170 und zur Abfuhr des Dialysats zu den Elementen 84, 80 und 86 der Dialysatabfuhr. Die Komponenten der Dialysataufbereitung umfassen typischerweise eine Wasseraufbereitungseinrichtung 42 zum Bereitstellen eines Stromes von aufbereitetem Wasser in die Dialysatzufuhrleitungen 62, 62', ein in einem Reservoir 44 bereitgestelltes Bicarbonat- Konzentrat, das mittels einer steuerbaren Bicarbonat-Pumpe 46 in dosierbarer Menge durch den Bicarbonat-Zufluss 48 in die Dialysatorzufuhrleitung 62 eindosiert wird, ein in einem Reservoir 54 bereitgestelltes Säure-Konzentrat, das mittels einer dosierbaren Säure-Pumpe 56 in dosierbarer Menge durch den Säure-Zufluss 58 in die Dialysatorzufuhrleitung 62 eindosiert werden kann, und eine stromabwärts der Zuflüsse 48, 58 in der Dialysatzufuhrleitung 62 in-line angeordnete Bilanzierungseinrichtung 60. Die Bilanzierungseinrichtung 60 oder eine in ihr enthaltene oder mit ihr verbundene Steuereinrichtung ist dazu ausgebildet, die Zusammensetzung und die abgegebene Menge, d.h. den Dialysatstrom, in einer auf den Patienten 10 abgestimmten und für den Fortgang der Dialysebehandlung spezifischen Weise zu steuern, und insbesondere dazu, die für diese Steuerung erforderlichen Steuersignale für die Bicarbonat-Pumpe 46, die Säure-Pumpe 56, eine stromabwärts der Bilanzierungseinrichtung 60 in der Dialysatzufuhrleitung 62' angeordnete Flusspumpe 64 für den Dialysatzugang und eine stromabwärts des Dialysatbereichs 170 in der Dialysatausflussleitung 82, 82' angeordnete Flusspumpe 84 zu erzeugen und die Flusspumpe 64 für den Dialysatzugang und die Flusspumpe 84 für die Dialysatabfuhr zu steuern. Das von der Flusspumpe 64 dosierte und stromauf derselben aufbereitete Dialysat wird durch das Dialysatoreingangsventil 72 und den Dialysateinlass 74 in den Dialysatbereich 170 der Dialysatorvorrichtung 100 geführt. Nach Durchführung des Stoffaustauschs mit dem Blut des Patienten 10 durch die Membran der Membranfiltereinrichtung 190 strömt das Dialysat weiter durch den Dialysatauslass 76 und wird durch das Dialysatorausgangsventil 78 dem Dialysatausfluss- bzw. abfuhrsystem 84, 80, 86 zugeführt. Das Dialysatabfuhrsystem umfasst eine typischerweise stromabwärts der Dialysatausfluss-Flusspumpe 84 in der Dialysatausflussleitung 82, 82' angeordnete Bilanzierungseinrichtung 80 und eine stromabwärts der Bilanzierungseinrichtung 80 in der Dialysatausflussleitung 82' angeordnete Dialysatabfuhr 86. Die Dialysatabfuhr 86 kann z.B. als ein ausleerbarer oder abführbarer bzw. entsorgbarer Auffangbehälter ausgebildet sein. In vielen Fällen kann das verbrauchte Dialysat aber auch direkt in den Abfluss und damit in die Wiederaufbereitung oder Kanalisation laufen. Der Dialysatkreislauf 40 umfasst ferner eine Bypass-Leitung 48 zu dem Dialysatbereich 170 der Dialysatorvorrichtung 100. Die Bypass- Leitung 48 kann mittels eines Bypass-Ventils 66 geöffnet bzw. geschlossen werden und ermöglicht einen Bypass von Dialysat bezüglich des Dialysatbereichs 170 vom Dialysatzugang 64 direkt zum Dialysatausfluss 84.

Im Einzelnen umfasst der extrakorporale Blutkreislauf 20 ein arterielles Schlauchsystem 24, einen Blutbereich 130 der Dialysatorvorrichtung 100 und ein venöses Schlauchsystem 34. Der extrakorporale Blutkreislauf 20 wird in einer Hämodialysebehandlung durch medizinisches Fachpersonal eingerichtet und umfasst ein arterielles Schlauchsystem 24 zum Ausführen von zu reinigendem Blut aus dem Patienten 10, den Blutbereich 130 der Dialysatorvorrichtung 100 und ein venöses Schlauchsystem 34 zum Rückführen von gereinigtem Blut zum Patienten 10. Das arterielle Schlauchsystem 24 umfasst einen arteriellen Zugang 22 zum arteriellen Blutgefäßsystem des Patienten 10, der beispielsweise als arterielle Nadel oder Katheter eingerichtet werden kann, und eine arterielle Blutpumpe 26 zum Pumpen von arteriellem Blut des Patienten 10 vom arteriellen Zugang 22 durch den Bluteinlass 28 in den Blutbereich 130 der Dialysatorvorrichtung 100 und weiter durch das venöse Schlauchsystem 34 zu einem venösen Zugang 36 in das venöse Blutgefäßsystem des Patienten 10. Die arterielle Blutpumpe 26 kann z.B. am oder im arteriellen Schlauchsystem 24 eingeschlaucht oder angeordnet sein. Das venöse Schlauchsystem 34 führt das im Dialysatbereich 130 gereinigte bzw. behandelte Blut von dem Blutauslass 32 des Dialysatbereichs 130 zu dem venösen Zugang 36 des Patienten 10 zurück. Dadurch wird der extrakorporale Blutkreislauf 20 geschlossen.

In der in Fig. 1 gezeigten schematischen Darstellung des Dialysatkreislaufs 40 und des Blutkreislaufs 20 findet, wie bereits erwähnt, der Stoffaustausch zwischen dem Dialysat und dem Blut des Patienten 10 im Dialysator bzw. in der Dialysatorvorrichtung 100 gemäß dem dritten Aspekt der Erfindung, genauer an der Membran der darin angeordneten Membranfiltereinrichtung 190 statt. Letztere unterteilt den Innenvolumenbereich 120 eines Gehäuses 110 der Dialysatorvorrichtung 100 in einen Dialysatbereich 170, der von dem über den Dialysatzulass 74 zuströmenden und durch den Dialysatabfluss 76 abgeführten Dialysat durchströmt wird, und den Blutbereich 130, der von dem durch den Bluteinlass 28 zuströmenden, zu reinigenden Blut des Patienten 10 und dem behandelten bzw. gereinigten und durch den Blutauslass 32 ab- und dem Patienten 10 zurückgeführten Blut durchströmt wird. Die Dialysatorvorrichtung 100 und/oder die darin angeordnete Membranfiltereinrichtung 190 umfasst eine passive oder eine aktive Identifizierungseinrichtung 192, die ein für die Identifizierung der Dialysatorvorrichtung 100 und/oder die Membranfiltereinrichtung 190 charakteristisches Identifizierungsmerkmal aufweist.

In einer Ausführungsform ist die passive Identifizierungseinrichtung 192 ein passiver RFID (Englisch: radio frequency identification) Chip, bei dem das Identifizierungsmerkmal durch Anstrahlen mit einer vorbestimmten elektromagnetischen Strahlung, deren Frequenz im z.B. RF (Englisch: radio frequency) Bereich liegt, auslesbar ist. In einer anderen Ausführungsform umfasst die passive Identifizierungseinrichtung 192 einen Code wie etwa einen Barcode oder ein Farbencodefeld, bei dem das Identifizierungsmerkmal durch Anstrahlen mit einer im optischen Bereich liegenden elektromagnetischen Strahlung mit einer Wellenlänge im Bereich des infraroten (IR), nahinfraroten, sichtbaren oder ultravioletten (UV) Lichts auslesbar ist.

An der in Fig. 1 gezeigten Dialysatorvorrichtung 100 gemäß dem dritten Aspekt der Erfindung ist eine Sensorvorrichtung 200 gemäß dem zweiten Aspekt der Erfindung lösbar und operativ verbindbar befestigt. Die Sensorvorrichtung 200 umfasst eine Signalempfangseinrichtung 210, die dazu ausgebildet ist, mindestens ein Signal, wie etwa ein Strahlungssignal oder Ultraschallsignal, von dem Außen- oder Randbereich oder auch aus dem Innenvolumenbereich 120 des Gehäuses 110 der Dialysatorvorrichtung 100 zu empfangen, und kann auch außen am Gehäuse angebracht oder als Bestandteil des Gehäuses ausgebildet sein. Das empfangene Signal ist charakteristisch für den Zustand bzw. die Identifizierung der Dialysatorvorrichtung 100 bzw. der darin angeordneten Membranfilereinrichtung 190. In einer Ausführungsform umfasst die Signalempfangseinrichtung 210 eine Identifizierungsleseeinrichtung 212, die dazu ausgebildet ist, einen für das Identifizierungsmerkmal der Identifizierungseinrichtung 192 indikativen Parameter der Strahlung zu detektieren und das Identifizierungsmerkmal und darüber die Identifizierung der Dialysatorvorrichtung 100 bzw. der Membranfiltereinrichtung 190 zu bestimmen. Damit das Signal aus dem Innenvolumenbereich 120 des Gehäuses 110 zu der außerhalb des Gehäuses 110 angeordneten Signalempfangseinrichtung 210 gelangen kann, ist das Gehäuse 110 für das Signal zumindest bereichsweise, oder als Ganzes, im Wesentlichen durchlässig.

Zum Anstrahlen der passiven Identifizierungseinrichtung 192 umfasst die Sensorvorrichtung 200 eine Strahlungsaussendeeinrichtung 240, die dazu ausgebildet ist, die vorbestimmte Strahlung, z.B. elektromagnetische Strahlung oder Ultraschallstrahlung, zum Bestrahlen zu der passiven Identifizierungseinrichtung 192 auszusenden, um diese dazu zu aktivieren, modifizierte elektromagnetische oder ultraschallakustische Strahlung, die das Identifizierungsmerkmal bzw. einen dafür indikativen Parameter enthält, auszusenden.

Wenn die passive Identifizierungseinrichtung 192 ein passiver RFID-Chip ist, umfasst die Strahlungsaussendeeinrichtung 240 eine RF-Strahlungsquelle (nicht gezeigt). In der Ausführungsform, wo die passive Identifizierungseinrichtung 192 ein Code wie beispielsweise ein Barcode, eine Datamatrix, allgemein ein 2-dimensionaler oder ein 3- dimensionaler Code, z.B. ein farbiger 20 Code, oder ein Farbencodefeld ist, umfasst die Strahlungsaussendeeinrichtung 240 eine z.B. optische oder akustische Strahlungsquelle zum Bestrahlen des Codes, und die Identifizierungseinrichtung 212 der Signalempfangseinrichtung 212 umfasst beispielsweise einen Codescanner zum Auslesen des Codes wie etwa einen Barcodescanner zum Lesen des Barcodes oder eine Kamera, beispielsweise mit einem zweidimensionalen CCD-Sensor oder einem 20-Codeleser, die dazu ausgebildet sind, die von dem Code, z.B. Barcode bzw. Farbencodefeld reflektierte Strahlung, z.B. optisches Licht zu detektieren, in geeigneter Weise zu analysieren und den in der detektierten Strahlung enthaltenen indikativen Parameter bzw. das Identifizierungsmerkmal zu erkennen.

In noch einer anderen Ausführungsform umfasst die Dialysatorvorrichtung 100 bzw. die Membranfiltereinrichtung 190 eine aktive Identifizierungseinrichtung 192, wie etwa einen aktiven RFID-Chip, die elektromagnetische Strahlung aussenden kann, die ein für die Identifizierung der Dialysatorvorrichtung 100 oder der Membranfiltereinrichtung 190 charakteristisches Identifizierungsmerkmal aufweist. Dabei ist es nicht erforderlich, in der Sensorvorrichtung 200 eine Strahlungsaussendeeinrichtung zum Anstrahlen der Identifizierungseinrichtung mit einer vorbestimmten elektromagnetischen Strahlung vorzusehen. Die Signalempfangseinrichtung 210 umfasst, wie in den Ausführungsformen mit einer passiven Identifizierungseinrichtung 192, eine Identifizierungsleseeinrichtung 212, die dazu ausgebildet ist, die von der (hier aktiven) Identifizierungseinrichtung 192, z. B. dem aktiven RFID-Chip, ausgesendete elektromagnetische Strahlung zu detektieren und, etwa nach einer Demodulationsauswertung, das Identifizierungsmerkmal zu erkennen.

In noch einer anderen Ausführungsform umfasst die Signalempfangseinrichtung 210 einen Empfänger für ein elektrisches Signal, das indikativ ist für eine zu messende Kapazität und/oder eine zu messende Induktivität, die charakteristisch für den Zustand bzw. die Identifizierung der Dialysatorvorrichtung 100 und/oder der Membranfiltereinrichtung 190 ist.

In noch einer anderen Ausführungsform kann die Signalempfangseinrichtung 210 dazu ausgebildet sein, Signale von aus dem Innenvolumenbereich 120 austretender Ultraschallstrahlung zu empfangen. Die Ultraschall-Strahlung kann in den Innenvolumenbereich 120 eingestrahlt werden und in dem Dialysatbereich 170 und/oder dem Blutbereich 130 reflektiert werden oder diese durchqueren. In dieser Ausführungsform umfasst die Signalaussendeeinrichtung 240 einen Emitter zum Aussenden von Ultraschall-Strahlung in den Innenvolumenbereich 120, etwa fokussiert in den Dialysatbereich 170 oder in den Blutbereich 130 oder in den Bereich der Membran der Membranfiltereinrichtung 190. In dem Innenvolumenbereich 120 von den dort in Flüssigkeit (Dialysat und/oder Blut) gelösten Stoffen und/oder von der Membran der Membranfiltereinrichtung 190 beeinflusste eingestrahlte Ultraschall-Strahlung kann in einer Reflexionsanordnung, in einer Transmissionsanordnung oder in einer Streuungsanordnung der für die Detektion der Ultraschall-Strahlung ausgelegten Signalempfangseinrichtung 210 in Bezug auf die Ultraschall-Strahlung aussendende Signalaussendeeinrichtung 240 angeordnet sein.

In einer Reflexionsanordnung ist die Richtung der zu detektierenden Strahlung, hier z.B. Ultraschall-Strahlung, im Wesentlichen entgegengesetzt, z.B. in einem Winkel von 180° oder nahezu 180°, in Bezug auf die Richtung der eingestrahlten Strahlung gerichtet. Die Reflektion kann auch unter einem anderen Winkel als 180 Grad zur Richtung der eingestrahlten Strahlung erfolgen, also z.B. in einer schrägen Richtung oder rechtwinkligen Richtung. In einer Transmissionsanordnung ist die Richtung der zu detektierenden Strahlung im Wesentlichen parallel, d.h. in einem Winkel von 0° oder nahezu 0°, zu der Richtung der eingestrahlten Strahlung gerichtet. Bei einer Streuungsanordnung ist die Richtung der zu detektierenden Strahlung unter einem von 0° und 180° im Wesentlichen unterschiedlichen Winkel, z.B. 90°, gerichtet. Die hierfür Ultraschall-Strahlung eingeführte Terminologie (Reflexions-, Transmissions- und Streuungsanordnung) von zu messender Strahlung in Bezug auf die eingestrahlte Strahlung wird hierin auch für optische elektromagnetische Strahlung verwendet.

Wenn die Gehäusewand 110 für optische elektromagnetische Strahlung, d.h. hierin für infrarote (IR), nahinfrarote, sichtbare oder ultraviolette (UV) Lichtstrahlung zumindest bereichsweise durchlässig ist, dann können mit der Sensorvorrichtung 200 gemäß dem zweiten Aspekt der Erfindung auch verschiedenartige optische Messungen direkt an der Dialysatorvorrichtung 100 ausgeführt werden, aus denen Information bezüglich des Zustands oder einer Eigenschaft, z. B. einer Stoffkonzentration im Dialysat oder Blut oder einer daraus abgeleiteten Größe, oder der Geschwindigkeit in den Fasern der Dialysatorvorrichtung, oder der Braunsehen Bewegung, oder der Dialysance der Dialysatorvorrichtung 100 oder der Membranfiltereinrichtung 190 abgeleitet werden können. Derartige optische Messungen können in Reflexions-, Transmissions- und Streu-Anordnungen und ferner auch in Brechungsanordnung einer für die Messung einer Lichtintensität ausgebildeten Signalempfangseinrichtung 210 in Bezug auf eine optische Strahlungsaussendeeinrichtung 240 durchgeführt werden. In einer Ausführungsform umfasst die Signalempfangseinrichtung 210 eine optische Strahlungsmesseinrichtung 220, die einen ersten optischen Strahlungsdetektor 220 und einen zweiten optischen Strahlungsdetektor 240 umfasst (siehe Fig. 2).

Die Sensorvorrichtung 200 gemäß dem zweiten Aspekt der Erfindung kann eine Strahlungsaussendeeinrichtung 240 umfassen, die dazu ausgebildet ist, Strahlung, wie etwa optische Strahlung (d.h. Licht) oder Ultraschall-Strahlung, in den Innenvolumenbereich 120 des Gehäuses 110 der Dialysatorvorrichtung 100 auszusenden. Entsprechend kann die Signalempfangseinrichtung 210 dazu ausgebildet sein, die Intensität von aus dem Innenvolumenbereich 120 austretender elektromagnetischer Strahlung oder Ultraschall- Strahlung, die von der Strahlungsaussendeeinrichtung 240 in den Innenvolumenbereich 120 eingestrahlt worden ist, zu messen. Die von der Signalempfangseinrichtung 210 gemessene Strahlung kann das Ergebnis einer für den Zustand der Dialysatorvorrichtung 200 charakteristischen Wechselwirkung der eingestrahlten Strahlung sein, die im Innenvolumenbereich 120 des Gehäuses 110 zwischen der Strahlung und einem oder mehreren der Folgenden stattfindet: dem Dialysat im Dialysatbereich 170, dem Blut im Blutbereich 130, einem in dem Dialysat und/oder in dem Blut enthaltenen Stoff, der Membranfiltereinrichtung 190, und einen in bzw. an der Membranfiltereinrichtung 190 festgehaltenen Stoff, der beispielsweise aus dem Blut bzw. dem Dialysat entstammt. Dabei ist die Wechselwirkung durch von der Strahlungsaussendeeinrichtung 214 in den Innenvolumenbereich 120 des Gehäuses 110 ausgesendete Strahlung hervorgerufen worden und ein Teil dieser Strahlung ist nach der Wechselwirkung aus dem Innenvolumenbereich 120 zu der Signalempfangseinrichtung 210 gelangt.

Eine für den Zustand der Dialysatorvorrichtung 100 bzw. des Bluts im Blutbereich 130 bzw. des Dialysats im Dialysatbereich 170 charakteristische Information liefernde optische Wechselwirkung kann eine der folgenden sein:
- Reflexion von der Strahlungsaussendeeinrichtung 240 ausgesendeter optischer Strahlung (d.h. Licht) an einer Grenzfläche zwischen der Wand 112 des Gehäuses 110, beispielsweise einem Fensterbereich 114, 116, 118 (siehe Fig. 2) und dem Dialysat oder dem Blut,
- Reflexion von von der Strahlungsaussendeeinrichtung 240 ausgesendeter optischer Strahlung an einer Grenzfläche zwischen der Membranfiltereinrichtung 190 und dem Dialysat oder dem Blut,
- Transmission von von der Strahlungsaussendeeinrichtung 240 ausgesendeter, optischer Strahlung mit einer Messwellenlänge, beispielsweise im infraroten Spektralbereich, die von einem in dem Dialysat und/oder in dem 'Blut enthaltenen Stoff absorbiert werden kann und die auf ihrem Weg zu der Strahlungsempfangseinrichtung 220 einen Strahlungslaufweg durch das Dialysat und/oder durch das Blut zurückgelegt hat,
- Emission von Lumineszenz- oder Fluoreszenzstrahlung durch einen in dem Dialysat und/oder in dem Blut enthaltenen Stoff, wobei eine entsprechende Lumineszenz- oder Fluoreszenzreaktion in diesem Stoff durch von der Strahlungsaussendeeinrichtung 240 ausgesendete optische Strahlung, z.B. UV Licht, hervorgerufen worden ist,
- Brechung von von der Strahlungsaussendeeinrichtung 240 ausgesendeter optischer Strahlung an einer Grenzfläche zwischen der Wand 112 des Gehäuses 110, z.B. einem Fensterbereich 114, 116, 118 (siehe Fig. 2) und dem Dialysat oder dem Blut, wobei die ausgesendete Strahlung z.B. unter einem zwischen 0° und 180° oder 0° und 90° liegenden Einfallswinkel auf die Grenzfläche auftrifft,
- Streuung von von der Strahlungsaussendeeinrichtung 240 ausgesendeter optischer Strahlung an einem in dem Dialysat oder in dem Blut enthaltenen Stoff, wobei dies auch z.B. eine so genannte dynamische Streuung, inkl. z.B. Raman-Streuung, von z.B. polychromatischem oder monochromatischem Licht wie etwa dichromatischer, polychromatischer oder monochromatischer Laserstrahlung einschließt; hierbei kann zur Auswertung einer
- dynamischen Streuung eine Analyse des Zeitverhaltens des gestreuten Lichts durchgeführt werden; die Messung ist hierbei nicht auf die Auswertung der Intensität beschränkt - auch die Phase der elektromagnetischen Strahlung und deren Zeitverhalten kann Informationen enthalten und zusätzlich oder anstelle der Intensitätserfassung erfasst und ausgewertet werden, dabei kann auch das zeitliche Abklingverhalten von Impulsen ausgewertet werden;
- Wechselwirkung von von der Strahlungsaussendeeinrichtung 240 ausgesendeter optischer Strahlung mit einer passiven Identifizierungseinrichtung 192, die an der Dialysatorvorrichtung 100 oder an der Membranfiltereinrichtung 190 angebracht ist, wie etwa einem Barcode- oder einem Farbencode-Feld, wobei die Identifizierungseinrichtung 192 ein für die Identifizierung der Dialysatorvorrichtung 100 oder der Membranfiltereinrichtung 190 charakteristisches Identifizierungsmerkmal aufweist. Derartige Anwendungen wurden oben beschrieben.

Mit Verweis nun auf die Figuren 2 und 3 kann die Sensorvorrichtung 200 gemäß dem zweiten Aspekt der Erfindung als ein Cliparm bzw. als eine Clipmanschette 220 ausgebildet sein, der bzw. die direkt an dem Gehäuse 110 der Dialysatorvorrichtung 100 lösbar und operativ verbindbar, beispielsweise aufclipbar, befestigt werden kann. In den in den Figuren 2 und 3 gezeigten Ausführungsformen umfasst die Sensorvorrichtung 200 zwei Cliparme bzw. Clipmanschetten 210, 210', die das beispielsweise zylinderförmige Gehäuse 110 umgreifen und zwischen sich aufnehmen können.

In einer Ausführungsform, wie in Fig. 2 gezeigt, kann ein Cliparm bzw. eine Clipmanschette 210 relativ zu einer in einer Längsrichtung 102 des Gehäuses 110 der Dialysatorvorrichtung 100 gemessenen Länge eine geringere Länge aufweisen, und entsprechend an dem Gehäuse 110 in der Längsrichtung 102 verschiebbar sein. In dieser Ausführungsform kann mit der Sensorvorrichtung 200 ein gewünschter Parameter in einer entlang der Längsrichtung 102 des Gehäuses 110 definierbaren Position oder auch, z.B. mit sequenziellen Messungen, an verschiedenen Positionen entlang der Längsrichtung 102 die Variation eines Parameters entlang der Längsrichtung 102 des Gehäuses 110 gemessen werden.

In einer alternativen Ausführungsform, wie in Fig. 3 gezeigt, kann ein Cliparm bzw. eine Clipmanschette 210 der Sensorvorrichtung 200 relativ zu der Längsrichtung 102 des Gehäuses 110 eine der Länge des Gehäuses 110 vergleichbare Abmessung aufweisen und entsprechend entlang der Längsrichtung 102 nur unwesentlich oder gar nicht verschiebbar ausgebildet sein.

Allgemein und auch insbesondere in den in den Figuren 2 und 3 gezeigten Ausführungsformen kann es vorteilhaft sein, wenn die Sensorvorrichtung 200 in einer oder mehreren, wiederholbaren bzw. wiedereinstellbaren, vorbestimmten relativen Positionen bzw. Orientierungen in Bezug auf die Dialysatorvorrichtung 100 an dieser angebracht werden kann. Zu diesem Zweck umfasst das Gehäuse 110 der Dialysatorvorrichtung 100 an seiner Außenoberfläche mindestens einen ersten Koppelbereich 110, oder z.B. zwei sich an dem Gehäuse 110 im Wesentlichen gegenüberliegend angeordnete erste Koppelbereiche 111 und 111', der/die an vorbestimmten Positionen an der Vorrichtung 100 angeordnet sind.

Entsprechend umfasst die Sensorvorrichtung 200 an einer dem Gehäuse 110 zugewandten Seite, z.B. in den Figuren 2 und 3 an einer Innenseite des Cliparms bzw. der Clipmanschette 210: einen zweiten Koppelbereich 211, oder z.B. zwei Koppelbereiche 211 und 211', die in einem mit der Vorrichtung 100 verbundenen Zustand der Sensorvorrichtung 200 im Wesentlichen kongruent zu dem bzw. den ersten Koppelbereich/en 111, 111' an dem Gehäuse 110 angeordnet sind. In der in Fig. 2 gezeigten Ausführungsform können an der Außenoberfläche des Gehäuses 110 mehrere in Längsrichtung 102 des Gehäuses 110, z.B. gleichmäßig beabstandete erste Koppelbereiche 111 vorgesehen sein. Diese ermöglichen, die Sensorvorrichtung 200 an verschiedenen vorbestimmten Positionen entlang der Längsrichtung 102 des Gehäuses 110 lösbar anzubringen. Die zweiten Koppelbereiche 211, 211' sind dazu ausgebildet, mit den ersten Koppelbereichen 111, 111' einzugreifen und zusammenzuwirken, so dass die Position der Sensorvorrichtung 200 in Bezug auf das Gehäuse 110 der Vorrichtung 100 wohl definiert und vorbestimmt ist.

In einer Ausführungsform umfassen die ersten Koppelbereiche 111, 111' und die zweiten Koppelbereich 211, 211'jeweils einander anziehende Magnete. In einer anderen Ausführungsform umfassen die ersten Koppelbereiche 111, 111' sogenannte weibliche Aufnahmeelemente und die zweiten Koppelbereiche 211,211' sogenannte männliche Steckelemente, die zur lösbaren Aufnahme in den weiblichen Aufnahmeelementen ausgebildet sind. Vorzugsweise sind dabei die weiblichen Aufnahmeelemente den ersten Koppelbereichen 111, 111' des Gehäuses 110 der Dialysatorvorrichtung 100 und die männlichen Steckelemente den zweiten Koppelbereichen 211, 211' der Cliparme bzw. Clipmanschetten 210, 210' der Sensorvorrichtung 200 zugeordnet.

Bei einem, mehreren oder allen Ausführungsbeispielen kann die Sensorvorrichtung 200 und/oder die Dialysatorvorrichtung 100 eine spezielle Form aufweisen, z.B. eine oder mehrere Einbuchtungen, Vertiefungen oder Vorsprünge am oder im Dialysatorgehäuse, die nur dort eine zentrierte Anbringung des Clips, d.h. der Sensorvorrichtung erlauben.

Alternativ oder zusätzlich zu der hierüber beispielhaft beschriebenen Ausgestaltung der lösbaren, operativen Verbindbarkeit der Sensorvorrichtung 200 an der Dialysatorvorrichtung 100 kann diese auch durch die folgenden, mechanisch lösbaren, insbesondere formschlüssig befestigbaren Mechanismen bewirkt werden:
- Die Sensorvorrichtung 200 umfasst einen Grundkörper (nicht gezeigt) und eine Clipeinrichtung mit mindestens einem Paar von an dem Grundkörper befestigten, elastisch-flexiblen Cliparmen bzw. Clipmanschetten oder an dem Grundkörper elastisch-flexibel angelenkten Cliparmen bzw. Clipmanschetten (nicht gezeigt), die dazu ausgebildet sind, zwischen sich das Gehäuse 110 bzw. einen Bereich des Gehäuses 110 aufzunehmen. Aufgrund ihrer elastisch-flexiblen Ausgestaltung oder Anlenkung können die Cliparme bzw. Clipmanschetten das Gehäuse 110 oder einen Teil des Gehäuses 110 clipartig umklammern.
- Die Sensorvorrichtung 200 umfasst mindestens ein Paar von Armen bzw. Manschetten, die jeweils einen distalen Endbereich mit einem dort vorgesehenen ersten Glied einer Haken- oder Eingreifeinrichtung aufweisen. Dabei sind die Arme bzw. Manschetten dazu ausgebildet, das Gehäuse 110 in einem Winkelbereich von mehr als 180°, vorzugsweise mehr als 270° und mehr bevorzugt mehr als 320° zu umgreifen. Ferner ist eine elastisch spannbare Spanneinrichtung mit zwei gegenüberliegenden Endabschnitten vorgesehen, an denen jeweils eine zum lösbaren Eingreifen mit einem ersten Glied der Haken- oder Eingreifeinrichtung ausgebildetes, zweites Glied bzw. Gegenglied der Haken- oder Eingreifeinrichtung vorgesehen ist. Dabei kann in einem elastisch gespannten Zustand der Spanneinrichtung ein jeweiliges zweites Glied am Endabschnitt der Spanneinrichtung mit einem ersten Glied an den Endbereichen der Arme bzw. Manschetten der Sensorvorrichtung 200 eingreifen, so dass die Arme bzw. Manschetten der Sensorvorrichtung 200 zusammen mit der elastisch gespannten Spanneinrichtung das Gehäuse 110 vollständig umringen bzw. umspannen. Optional kann die Sensorvorrichtung 200 einen Grundkörper (nicht gezeigt) umfassen, an dem die Arme bzw. Manschetten angelenkt sind oder mit dem Arme bzw. Manschetten elastisch-flexibel verbunden sind.
- Die Sensorvorrichtung 200 ist dazu ausgebildet, z.B. in einer Vertiefung, in bzw. an der Wand 112 des Gehäuses 110 der Dialysatorvorrichtung 100 lösbar integriert zu werden. Diese Ausführungsform ist insbesondere dann geeignet, wenn die' Sensorvorrichtung 200 eine räumlich kleine Ausdehnung aufweist, wie etwa im Fall einer RF-Identifizierungsausleseeinrichtung 212.

In der in Fig. 2 gezeigten Ausführungsform eines Systems gemäß dem ersten Aspekt der Erfindung mit einer Sensorvorrichtung 200 gemäß dem zweiten Aspekt der Erfindung ist das Gehäuse 110 der Dialysatorvorrichtung gemäß dem dritten Aspekt der Erfindung für optische Strahlung (d.h. Licht) nicht ausreichend durchlässig bzw. transparent. Daher sind in der Wand 112 des Gehäuses 110 sogenannte Fensterbereiche 114, 116, 118 vorgesehen, die für optische Strahlung ausreichend durchlässig bzw. transparent sind. In der Clipmanschette 210 ist eine Strahlungsaussendeeinrichtung 240 integriert, die eine erste, z.B. optische oder sonstige, Strahlungsquelle 242 zum Aussenden von elektromagnetischer Strahlung, z.B. Licht, in den Blutbereich 130 und eine zweite, z.B. optische, Strahlungsquelle 244 zum Aussenden von elektromagnetischer Strahlung, z.B. Licht in den Dialysatbereich 170 umfasst. Integriert in den Cliparm bzw. die Clipmanschette 210, 210' ist ferner eine Strahlungsmesseinrichtung 220 mit einem ersten, z.B. optischen Strahlungsdetektor, der zum Detektieren von aus dem Blutbereich 130 austretender elektromagnetischer Strahlung, z.B. Licht, ausgebildet ist, und einem zweiten, z.B. optischen, Strahlungsdetektor 224, der zum Detektieren von aus dem Dialysatbereich 170 austretender elektromagnetischer Strahlung, z.B. Licht, ausgebildet und entsprechend angeordnet ist. Dabei ist der erste Strahlungsdetektor 222 dazu ausgebildet, aus dem Blutbereich 130 austretender elektromagnetischer Strahlung, z.B. Licht zu messen, das von der ersten Strahlungsquelle 242 dort hinein gestrahlt worden ist. Der zweite Strahlungsdetektor 224 ist dazu ausgebildet, aus dem Dialysatbereich 170 austretende elektromagnetische Strahlung, z.B. Licht zu detektieren, die von der zweiten Strahlungsquelle 244 dort hinein gestrahlt worden ist.

In einer Ausführungsform ist der erste Strahlungsdetektor 222 in Bezug auf die erste Strahlungsquelle 242 und den Blutbereich 130 so räumlich angeordnet bzw. positioniert, dass das von der ersten Strahlungsquelle 242 in den Blutbereich 130 ausgestrahlte Licht von dem zweiten Strahlungsdetektor 222 in einer Reflexionsanordnung, in der nur im Wesentlichen zurückgeworfenes bzw. rückwärts gestreutes Licht gemessen wird. In einer anderen Ausführungsform ist der erste Strahlungsdetektor 222 in Bezug auf die erste Strahlungsquelle 242 und den Blutbereich 130 in einer Transmissionsanordnung angeordnet, in der im Wesentlichen nur Licht gemessen wird, das Streuung in einer im Wesentlichen Vorwärtsrichtung oder Transmission in Richtung eines wohl definierten Laufwegs durch den Blutbereich 130 untergangen hat. In noch einer anderen Ausführungsform ist eine Streuanordnung realisiert, bei der ein Blickwinkel des ersten Strahlungsdetektors 222 in Bezug auf eine Hauptabstrahlrichtung der ersten Strahlungsquelle 242 unter einem Winkel, vorzugsweise einem Winkel von etwa 90°, jedenfalls einem Winkel der wesentlich von 0° und 180° verschieden ist, gemessen wird. Jeweils entsprechendes gilt für den zweiten optischen Strahlungsdetektor 224 und dessen Anordnung bzw. Position in Bezug auf die zweite Strahlungsquelle 244 und den Dialysatbereich 170.

In der in Fig. 3 gezeigten Ausführungsform eines Systems gemäß dem ersten Aspekt der Erfindung ist die Sensorvorrichtung 200 gemäß dem ersten Aspekt der Erfindung so ausgebildet, dass sie das im Beispiel zylinderförmige Gehäuse 110 der Dialysatorvorrichtung 100 gemäß dem dritten Aspekt der Erfindung nahezu vollständig umgreift und überdeckt. Dies ist im Beispiel der Fig. 3 durch eine Ausbildung der Sensorvorrichtung 200 in Form einer Clipmanschette 210, 210' realisiert.

Im rechten Bereich der Fig. 3 ist die geöffnete, von dem Gehäuse 110 abgenommene und planar entrollte Innenseite der Sensorvorrichtung 200 gezeigt, die in einem an der Dialysatorvorrichtung 100 angebrachten Zustand dieser Vorrichtung 100 zugewandt ist. In der im Wesentlichen rechteckförmigen innenseitigen Fläche der Sensorvorrichtung 200 ist eine Vielzahl von Strahlungseintrittsbereichen 230, 232-x, 232-y, 236-x,y und eine Vielzahl von Strahlungsaustrittsbereichen 250, 252-x, 254-y, 256-x,y ausgebildet. Zur Bezeichnung und Definition von Richtungen ist für die entrollte Sensorvorrichtung 200 in Fig. 3 die Richtung der Längsachse 102 und die Richtung des Umfangs 104 des Gehäuses 110 der Dialysatorvorrichtung 100 mit gestrichelten Linien eingezeichnet.

In einer Ausführungsform bildet die Vielzahl der Strahlungseintrittsbereiche 230 eine z. B. in der Richtung des Umfangs 104 ausgerichtete, eindimensionale Anordnung 231 von Strahlungseintrittsbereichen 232-x, die sich über einen Teil des Umfangs oder bei einem oder mehreren Ausführungsbeispielen auch im Wesentlichen über die gesamte Ausdehnung entlang der Umfangsrichtung 104 der Sensorvorrichtung 200 erstrecken kann. In einer dazu alternativen oder zusätzlich realisierbaren Ausführungsform bildet eine Vielzahl der Strahlungseintrittsbereiche 230 eine zweite eindimensionale Anordnung 233 mit einer Vielzahl von Strahlungseintrittsbereichen 234-y, die sich über einen Teil oder bei einem oder mehreren Ausführungsbeispielen im Wesentlichen über die gesamte Ausdehnung der Sensorvorrichtung 200 entlang der Richtung der Längsachse 102 erstrecken kann. In einer weiteren Ausführungsform bilden die Strahlungseintrittsbereiche 230 eine zweidimensional e Anordnung 235 mit einer Vielzahl von Strahlungseintrittsbereichen 236-x,y, die in einer schräg oder parallel zu der Richtung des Umfangs 104 und der Richtung der Längsachse 102 ausgerichteten regelmäßigen Anordnung, etwa einer rechtwinkligen Gitteranordnung, oder auch in unregelmäßiger Form angeordnet sein können. Dabei erstreckt sich diese zweidimensionale Anordnung 235 bzw. Gitteranordnung über einen Teil der, oder bei einem oder mehreren Ausführungsbeispiel en auch im Wesentlichen über die gesamte, Oberfläche der Sensorvorrichtung 200. Die Anordnung der Strahlungseintrittsbereiche muss sich nicht über die gesamte Sensorvorrichtung erstrecken, sondern kann auch nur in Teilbereichen vorhanden sein, die von besonderem Interesse sind.

In jedem Strahlungseintrittsbereich 230, 232-x, 232-y, 236-x,y ist entweder ein Strahlungsempfänger, insbesondere ein Lichtdetektor angeordnet, oder ein Eintrittsbereich eines optischen Lichtleiters bzw. einer Lichtleitfaser. Im letzten Fall leitet der Lichtleiter bzw. die Lichtleitfaser das in dem Strahlungseintrittsbereich in den Lichtleiter bzw. die Lichtleitfaser eingetretene Licht durch den Lichtleiter bzw. die Lichtleitfaser bis zu einem Strahlungsaustrittsbereich am entgegengesetzten Ende, wo ein Lichtdetektor vorgesehen ist, der das aus dem entgegengesetzten Ende des Lichtleiters bzw. der Lichtleitfaser austretende Licht detektiert. Jeder Lichtdetektor kann, je nach Messaufgabe und zu erwartender Lichtintensität bzw. Wellenlänge einer der folgenden sein: eine Fotodiode, ein Fototransistor, ein CMOS-Lichtdetektor, ein Fotomultiplier oder eine Avalanche-Fotodiode. In der Ausführungsform mit Lichtleitern bzw. Lichtleitfasern können die Lichtaustrittsenden zu einer zu der Anordnung der Lichteintrittsöffnungen kongruenten Gitteranordnung zusammengefasst sein und das aus der Vielzahl dieser Austrittsöffnungen auftretende Licht auf einem entsprechend angeordneten bzw. ausgerichteten ein- oder zweidimensionalen Lichtdetektor, beispielsweise einem ein- oder zweidimensionalen CCD-Sensor, in seiner räumlichen Verteilung und bezüglich seiner Intensität gemessen werden.

Entsprechend der räumlichen Anordnung der Strahlungseintrittsbereiche 230 in eindimensionalen Anordnungen 231, 233 oder eine zweidimensionale Anordnung 235 kann auch die Vielzahl der Strahlungsaustrittsbereiche 250 in einer der folgenden Anordnungen ausgebildet sein: eine eindimensionale Anordnung 251 von Strahlungsaustrittsbereichen 252-x, die entlang der Richtung des Umfangs 104 verteilt angeordnet sind und sich im Wesentlichen über die gesamte Ausdehnung der entrollten Sensorvorrichtung 200 erstrecken, eine eindimensionale Anordnung 253 von Strahlungsaustrittsbereichen 254-y, die entlang der Richtung der Längsachse 102 verteilt angeordnet sind und sich im Wesentlichen über die gesamte Ausdehnung in dieser Richtung auf der entrollten Sensorvorrichtung 200 erstrecken, und/oder eine zweidimensionale Anordnung 255 von einer Vielzahl von Strahlungsaustrittsbereichen 256-x,y, die in einer entlang der Richtung des Umfangs 104 und der Längsachse 102 orientierten, im Wesentlichen rechtwinkligen Gitteranordnung angeordnet sind. In jedem Strahlungsaustrittsbereich 250 kann entweder eine Lichtquelle, wie etwa eine LED, oder eine Austrittsöffnung für einen Austritt von Licht aus einem/r Licht zuführenden Lichtleiter bzw. Lichtleitfaser angeordnet sein. In der Ausführungsform mit Licht zuführenden Lichtleitern bzw. Lichtleitfasern kann am gegenüberliegenden Ende eines/r jeweiligen Lichtleiters bzw. Lichtleitfaser eine gesonderte Lichtquelle angeordnet sein. Alternativ dazu können die Strahlungsaustrittsbereiche 250 auch in einer beispielsweise zu der Gitterstruktur der Strahlungseintrittsbereiche 230 kongruenten Gitteranordnung zusammengefasst sein und von mehreren Strahlungsquellen oder auch einer einzigen Lichtquelle, die z.B. ein homogenes Strahlungsfeld bzw. paralleles Licht abstrahlt, angestrahlt werden, wie etwa ein aufgeweiteter Laserstrahl. Auch andere Lichtquellen können verwendet werden, wie z.B. eine oder mehrere Halogenlampen oder sonstige Lampen, Fluoreszenzlichtquellen oder dgl. Wenn mehrere Lichtquellen verwendet werden, sind die Lichtquellen bei einem, mehreren oder allen Ausführungsbeispielen einzeln schaltbar, so dass eine gezielte, gesteuerte Bestrahlung durchführbar ist.

Durch selektive Ansteuerung bzw. Strahlungsemission aus den Strahlungsaustrittsbereichen 250 können, wie ein Fachmann leicht erkennt, unterschiedliche Lichteinstrahlungsverteilungen bzw. Beleuchtungsgeometrien realisiert werden. Auch können durch selektive Messung von Strahlungsintensitäten in selektiv ausgewählten Strahlungseintrittsbereichen 230 verschiedenartige Abstrahl- bzw. Ausstrahlkonfigurationen von Licht aus dem Gehäuse 110 der Dialysatorvorrichtung 100 realisiert werden.

In der Ausführungsform, bei der Strahlung von den Strahlungseintrittsbereichen durch einen Lichtleiter bzw. eine Lichtleitfaser zu einem räumlich entfernten Detektor geleitet wird, kann hinter dem Lichtaustrittsbereich aus Lichtleiter bzw. Lichtleitfaser auch eine Lichtmesseinrichtung vorgesehen sein, die eine Wellenlängenauswahleinrichtung, wie etwa einen Monochromator, und einen dahinter angeordneten Lichtdetektor umfassen kann. Die Wellenlängenauswahleinrichtung kann dazu ausgebildet sein, einen schmalen Wellenlängenbereich, der eine vorbestimmte Nachweis- bzw. Messwellenlänge umfasst, auszuwählen.

Beispielsweise kann ein schmalbandiger Wellenlängenbereich von Messwellenlängen so realisiert bzw. abgetastet werden, dass ein Lumineszenz- oder Fluoreszenzemissionsspektrum erfasst werden kann. Ein Fachmann weiß, dass ein Lumineszenz-, Fluoreszenz- oder Fluoreszenzemissionsspektrum charakteristisch ist für einen ausgewählten Stoff, der beispielsweise im Blutbereich 130 bzw. im Blut gelöst ist, oder der im Dialysatbereich 170 bzw. im Dialysat gelöst ist. Hierbei kann bei einem oder mehreren Ausführungsbeispielen auf dem Gehäuse ein solches lumineszierendes oder fluoreszierendes Material angebracht sein, das zur Identifikation des Dialysators dienen kann. Damit kann der Typ des gerade eingesetzten, verwendeten oder zu verwendenden Dialysators erkannt werden.

In Fig. 4 ist veranschaulicht, wie eine Dialysatorvorrichtung 100_ gemäß dem dritten Aspekt der Erfindung und eine Sensorvorrichtung 200 gemäß dem zweiten Aspekt der Erfindung in Bezug auf die Steuerung bzw. Auslesung und Führung von Mess- und Steuersignalen in einer Dialysemaschine 300 gemäß dem vierten Aspekt der Erfindung integriert bzw. an dieser angeschlossen sein kann. Die in Fig. 4 gezeigte Dialysemaschine 300 umfasst die in Fig. 1 gezeigten Komponenten des Dialysatkreislaufs 40 und des Blutkreislaufs, die in Fig. 4 nicht im Einzelnen dargestellt sind.

Die Dialysatorvorrichtung 100 ist über den in Fig. 1 gezeigten Dialysatzufluss 72 und den Dialysatabfluss 78 fluidtechnisch in den Dialysatkreislauf 40 der Dialysemaschine 300 eingebunden. Ferner ist die Dialysatorvorrichtung 100 über das arterielle Schlauchsystem 24 und das venöse Schlauchsystem 34 mit dem arteriellen Zugang 22 und dem venösen Zugang 36 des Patienten 10 verbunden, siehe Fig. 1.

Die in Fig. 4 gezeigte Dialysemaschine 300 umfasst ein Maschinengehäuse 302, in dem die in Fig. 1 gezeigten Komponenten des Dialysatkreislaufs 40 angeordnet sind, ausgenommen die Dialysatorvorrichtung 100. Diese kann innerhalb des Maschinengehäuses 302 oder extern dazu angeordnet sein. Bei externer Anordnung ist die Dialysatorvorrichtung 100 über die Dialysatzufuhr 72 und den Dialysatablass 78 in Fluidkommunikation mit den innerhalb des Maschinengehäuses 302 angeordneten Komponenten des Dialysatkreislaufs 40, siehe Fig. 1. Das Maschinengehäuse 302 umfasst Gehäuserollen 304, 304', mittels der die Maschine 300 auf einem Boden rollbar ist. Auf dem Maschinengehäuse 302 angeordnet oder beabstandet dazu und mit den entsprechenden Datenleitungen angeschlossen bereitgestellt ist eine Datenausgabe- bzw. Visualisierungseinrichtung 310 und eine Dateneingabeeinrichtung (nicht gezeigt). Die Dateneingabeeinrichtung und Datenausgabe- bzw. Visualisierungseinrichtung 310 zubilden zusammen eine Eingabe-/Ausgabeeinrichtung, die in jeglicher hinsichtlich ihrer Vielfältigkeit dem Fachmann bekannten Formen moderner Mensch-Maschine-Schnittstellen ausgestaltet sein kann.

Die Dialysemaschine 300 umfasst auch eine Steuereinheit 320, die zum Steuern der Komponenten des Dialysatkreislaufs 40, der Strahlungsaussendeeinrichtung 210 und deren Komponenten sowie zur Steuerung der Strahlungsmesseinrichtung 240 und deren Teilkomponenten ausgebildet ist, eine Datenverarbeitungs- bzw. Auswerteeinheit 330, die dazu ausgebildet ist, die von der Sensorvorrichtung 200 erzeugten Messsignale aufzunehmen, umzurechnen in relevante Parameter, auszuwerten und zur Speicherung bzw. Datensicherung bereitzustellen, und eine Speichereinheit 340, die dazu ausgebildet ist, von der Datenverarbeitungs- bzw. Auswerteeinheit 330 bereitgestellte Messdaten bzw. ausgewertete Informationen aufzunehmen und zu speichern und insbesondere ferner Computer-Programme, die die Steuereinheit 320, die Datenverarbeitungs- bzw. Auswerteeinheit 330 und/oder die Eingabe-/Ausgabeeinrichtung 210 steuern, zu speichern.

Die Dialysemaschine 300 umfasst ferner ein Datenbussystem 360, an dem die steuerbaren Komponenten des Dialysatkreislaufs 40 und des Blutkreislaufs 20, die in Fig. 4 gezeigten Einheiten 3.10, 320, 330, 340 sowie die Dialysatorvorrichtung 100 und die daran lösbar anbringbare Sensorvorrichtung 200 angeschlossen und kommunikationstechnisch miteinander verbunden sind. Das Datenbussystem 360 umfasst eine Eingabe-/Ausgabedatenleitung 362, mittels der die Eingabe-/Ausgabeeinrichtung 310 kommunikationstechnisch angeschlossen ist, eine Steuerleitung 364, mittels der die Steuereinheit 320 kommunikationstechnisch angeschlossen und in die Lage versetzt ist, Daten von der Eingabe-/Ausgabeeinrichtung 310 zu empfangen und Steuerdaten bzw. ausgewertete Daten zu der Eingabe-/Ausgabeeinrichtung 310, etwa zur Ausgabe bzw. Anzeige, zu übertragen, eine Betriebsdatenleitung 366, mittels der die Steuereinheit 360 und die Datenverarbeitungs- bzw. Auswerteeinheit 330 kommunikationstechnisch miteinander verbinden sind, eine Datenspeicherleitung 368, mittels der die Speichereinheit 340 und die Datenverarbeitungs- bzw. Auswerteeinheit 330 kommunikationstechnisch miteinander verbinden sind, eine Dialysatorvorrichtungssignalleitung 370, mittels der die Dialysatorvorrichtung 100 und die Dialysemaschine 300 kommunikationstechnisch miteinander verbunden sind, und eine Sensorvorrichtungssignalleitung 372, mittels der die Sensorvorrichtung 200 und die Datenverarbeitungs- bzw. Auswerteeinheit 330 Dialysemaschine 300 kommunikationstechnisch miteinander verbunden sind.

Die Dialysatorvorrichtung 100 ist mittels der Dialystorvorrichtungssignalleitung 370 mit der Steuereinheit 320 und die an der Dialysatorvorrichtung 100 lösbar anbringbare Sensorvorrichtung 200 mittels der Sensorvorrichtungssignalleitung 372 mit der Datenverarbeitungs- bzw. Auswerteeinheit 330 der Dialysemaschine 300 kommunikationstechnisch verbunden. Über die Sensorvorrichtungssignalleitung 372 kann die Datenverarbeitungs- bzw. Auswerteeinheit 330 von der Sensorvorrichtung 200 erzeugte Messdaten, z.B. gemessene Strahlungsintensitätsdaten oder Identifizierungsdaten, zu der Datenverarbeitungs- bzw. Auswerteeinheit 330 zur Verarbeitung und Auswertung übertragen und auch von der Einheit 330 Steuerungsdaten zum Ansteuern der Strahlungsaussendeeinrichtung/en 240 empfangen. Über die Dialysatorvorrichtungssignalleitung 370 kann die Dialysatorvorrichtung 100 von der Steuereinheit 320 Steuersignale empfangen und umgekehrt für ihren Zustand bzw. ihre Identifizierung charakteristische Information an die Steuereinheit 320 übertragen.

Die in Fig. 4 gezeigte Dialysemaschine 300 umfasst ferner einen ersten Gehäusehalter 180 und einen zweiten Gehäusehalter 182. Diese sind dazu ausgebildet, zwischen sich die Dialysatorvorrichtung 100 lösbar einsetzbar zu tragen. Der erste Gehäusehalter 180 und ggf. der zweite Gehäusehalter 182 können als Endkappe zum lösbaren Aufsetzen auf ein jeweiliges Ende der Dialysatorvorrichtung 100 ausgebildet und dabei insbesondere wiederverwendbar sein, während die Dialysatorvorrichtung 100 als Ganzes als Einmalartikel ausgebildet ist. Dabei dienen die Gehäusehalter 180, 182 zur fluidtechnischen Einbindung der Dialysatorvorrichtung 100 in den Dialysatkreislauf 40 und den Blutkreislauf 20. Die Sensorvorrichtung 200 kann mit dem ersten oder zweiten Gehäusehalter 180, 182 dauerhaft verbunden sein oder auch getrennt hiervon ausgebildet sein.

In einer Ausführungsform ist der erste Gehäusehalter 180 fest mit dem Gehäuse 220 der Dialysemaschine 300 verbunden, und der zweite Gehäusehalter 182 ist als gesondertes Teil ausgebildet und an der Dialysatorvorrichtung 100 lösbar, z.B. anclipbar, und ebenso an dem Maschinengehäuse 302 lösbar, z.B. anclipbar ausgebildet. In einer anderen Ausführungsform ist nur ein einziger Gehäusehalter 180 vorgesehen, der die Dialysatorvorrichtung 100 lösbar, z.B. anclipbar, und zuverlässig trägt. In einer noch anderen Ausführungsform werden die Dialysatorvorrichtung 100 und die Gehäusehalter 180 und 182 abgesetzt von der Dialysemaschine 300, beispielsweise in der Nähe des zu behandelnden Patienten 10, eingesetzt.

Es ist für einen Fachmann offensichtlich, dass die Sensorvorrichtung 200, insbesondere in den bezüglich der Figuren 2 und 3 beschriebenen Ausführungsformen als Ganzes so ausgebildet sein kann, dass die folgenden Funktionen bzw. konkretisierten Ausgestaltungen optischer Messungen an der Dialysatorvorrichtung 100 durchgeführt werden können. Konkret kann die Funktionalität der in Fig. 3 gezeigten Sensorvorrichtung 200 z.B. für folgende Aufgaben angepasst werden:
(i) Messungen im Blutbereich 130 zur Bestimmung der Konzentrationen von Stoffen wie etwa von Albumin, anderen Stoffen oder urämischen Toxinen im Blut,
(ii) Messung von physikalischen Parametern des Bluts im Blutbereich 130 bzw. des Dialysats im Dialysatbereich 170, z.B. die Viskosität und/oder die Strömungsgeschwindigkeit des Dialysats bzw. des Bluts oder die Hämatokritkonzentration im Blut, und
(iii) Messungen im Dialysatbereich 170 zur Bestimmung der Konzentration von Stoffen wie etwa von Proteinen, anderen Stoffen oder urämischen Toxinen im Dialysat.

Im Folgenden werden Ausführungsbeispiele für mit der Sensoreinrichtung 200 ausführbare optische Messungen beschrieben.

### 1. Messungen an der Dialysatorvorrichtung

In die mit Verweis auf die Fig. 2 beschriebene Sensoreinrichtung 200 eingebaut oder über Lichtleitfasern bzw. Lichtleiter optisch damit verbunden ist eine Lichtquelle vorgesehen, die schmalbandiges Licht aussendet, z.B. eine LED, ein Laserstrahl oder ein/e, monochromatisiertes Licht führende/r Lichtleiter bzw. Lichtleitfaser. Diese Lichtquelle strahlt, eventuell über ein strahlformendes Element, z.B. eine Sammellinse, senkrecht oder in einem vorbestimmten Winkel durch eine transparente Stelle, z.B. durch einen Fensterbereich 114, 116, 118 Licht in den Innenvolumenbereich 120 des Gehäuses 110 ein. Das eingestrahlte Licht. trifft in den Bereich der Dialysatorvorrichtung 100, in dem die Membran der Membranfiltereinrichtung 190 mit ihren Membranfasern angeordnet ist. Räumlich benachbart zu der Lichtquelle in der Sensorvorrichtung 200 integriert oder über eine/n Lichtleiter bzw. Lichtleitfaser optisch damit verbunden ist ein Lichtdetektor, der in seinem Blickfeld gestreutes oder reflektiertes Licht einfängt, das z.B. an dem Gehäuse, an der Grenzfläche zwischen dem Gehäuse und der Flüssigkeit oder in dem von der Lichtquelle bestrahlten Bereich des Innenvolumenbereichs 120 reflektiert wird. Der Lichtdetektor kann dabei eine einzelne oder mehrere Photodioden oder auch ein anderer Detektortyp, wie etwa ein Phototransistor, ein CMOS-Detektor, ein CCD, ein Photomultiplier oder eine Avalanche-Photodiode sein.

Verteilt über der Innenseite der Sensorvorrichtung 200 angeordnet ist eine eindimensionale Anordnung 231, 233 oder eine zweidimensionale Anordnung 235 von Lichtdetektoren bzw. Lichteintrittsöffnungen (Strahlungseintrittsbereichen 230) mit den Eintrittsenden von Lichtleitern bzw. Lichtleitfasern, deren Lichteintrittfacetten in die Blickrichtung zeigen, aus der die zu analysierende Lichtstrahlung kommt. Die Lichtleiter bzw. Lichtleitfasern dienen dazu, das zu analysierende Licht aus der Sensorvorrichtung 200 herauszuleiten und an anderer Stelle z.B. mittels der vorgenannten Lichtdetektoren, gegebenenfalls in Kombination mit Wellenlängenauswahleinrichtungen (z.B. Monochromator/en), zu analysieren. Durch Auswahl der Messwellenlänge lässt sich einstellen, mit welchen im Dialysat und/oder im Blut gelösten Stoffen, das analysierte Licht wechselgewirkt hat. Der Eintritt und/oder Austritt des Lichts kann auch unter einem Winkel zur Gehäuseoberfläche erfolgen, wobei die Lichtdetektoren oder Lichtleiter hierbei schräg zur Gehäuseoberfläche angeordnet sein können.

Beispielsweise findet im grünen Spektralbereich (500 - 600 nm) die Wechselwirkung von Licht vor allem mit dem Hämoglobin im Blut statt. Hämoglobin weist in diesem Spektralbereich Absorptionsbanden auf. Im infraroten Spektralbereich absorbiert z.B. im Blut enthaltene Glucose (etwa bei der Wellenlänge 2,3 µm) und Harnstoff (etwa bei einer Wellenzahl (reziproke Wellenlänge) im Bereich von ca. 4500 bis 4700 cm·1

In den Strahlungseintrittsbereichen 230 der Sensorvorrichtung 200 können auch eine oder mehrere Strahlungsmesseinrichtungen 220 mit einer eine Fokus- bzw. Fokalrichtung aufweisenden Messoptikeinrichtung und einem eine lichtsensitive Detektorfläche aufweisenden Lichtdetektor angeordnet sein. Dabei kann die Messoptikeinrichtung dazu ausgebildet sein, einen Raumbereich aus dem Innenvolumenbereich 120, z.B. aus dem Blutbereich 130 oder dem Dialysatbereich 170, auf die Detektorfläche des Lichtdetektors abzubilden, wobei der abgebildete Raumbereich durch Einstellen der Fokaltiefe und der Fokalrichtung definiert ist. Durch Änderung der Fokaltiefe und gegebenenfalls zusätzlich durch Änderung der Fokaltiefe des detektierten Lichts kann der Bereich, in dem eine optische Wechselwirkung stattfindet, im Innenvolumenbereich 120 ausgewählt werden.. Durch Auswahl der Messwellenlänge kann ein spezifischer nachzuweisender Analyt ausgewählt werden.

Bei den Wechselwirkungen Reflexion, Absorption und Transmission gleicht die Wellenlänge des analysierten Lichts der Wellenlänge der eingestrahlten Lichtstrahlung. Die Lichtstrahlung kann bei einem oder mehreren Ausführungsbeispielen im Wesentlichen senkrecht auf die Gehäusewand 112 der Dialysatorvorrichtung 100 eingestrahlt und in der gleichen Richtung, oder auch rechtwinklig (oder nahezu rechtwinklig) dazu analysiert werden, z.B. bei Ausnutzung von Fluoreszenzeigenschaften. In dieser Messanordnung lässt sich aus einer geringen analysierten Lichtintensität auf das Vorliegen einer starken Absorption schließen, was wiederum Rückschlüsse auf die Konzentration eines z.B. Absorption verursachenden Stoffes oder einer Stoffmischung ermöglicht. So kann eine zeitliche Veränderung und eine räumliche Verteilung der Absorption erfasst und damit eine räumliche Verteilung und/oder eine zeitliche Veränderung einer Stoffkonzentration gemessen werden.

### 2. Fluoreszenz- bzw. Lumineszenzmessungen an der Dialysatorvorrichtung

Ist die Wellenlänge der analysierten Lichtstrahlung eine andere, z.B. eine längere.• als die Wellenlänge der eingestrahlten Lichtstrahlung, so können mit der zuvor beschriebenen Messanordnung, mit einer Streuanordnung der Blickrichtung des Lichtdetektors in Bezug auf die Einstrahlrichtung der Lichtquelle, bei entsprechender Intensität und Auswahl der Wellenlänge der eingestrahlten Lichtstrahlung, z.B. im ultravioletten (UV) Spektralbereich, Fluoreszenz- bzw. Lumineszenzwechselwirkungen mit im Blut oder im Dialysat gelösten Stoffen induziert, gemessen und gegebenenfalls auch quantifiziert werden. Dabei ist es bei einem oder mehreren Ausführungsbeispielen möglich, die die Wechselwirkung anregende Lichtstrahlung in zeitlich kurzen, z.B. hoch-intensiven Lichtpulsen einzustrahlen und das infolge der Fluoreszenz- bzw. Lumineszenzreaktion erzeugte Licht zeitlich und/oder spektral aufgelöst zu detektieren. Derartige Fluoreszenz- bzw. Lumineszenzmessungen ermöglichen Rückschlüsse auf im Dialysat bzw. im Blut gelöste Stoffe sowie deren Konzentration und deren räumliche Verteilung. Dies ermöglicht eine zeitaufgelöste Messung, bei der die Abklingzeit der Fluoreszenz als Charakteristikum gemessen wird.

Anstelle von Pulsen kann auch eine kontinuierliche Bestrahlung vorgesehen sein.

Der Detektor ist bei einem oder mehreren Ausführungsbeispielen in einem Winkel, z.B. in einem rechten Winkel, zur Lichteinfallsrichtung angeordnet, so dass die Messungen durch Reflektionen kaum oder gar nicht gestört werden.

### 3. Brechung von Lichtstrahlung an der Dialysatorvorrichtung

Die möglichen Ausgestaltungen der Lichteinstrahlung und der Lichtdetektion aus dem Beispiel 1 gelten hier entsprechend. Konkret lässt sich damit eine Messung von Lichtbrechung an der Dialysatorvorrichtung 100 wie folgt realisieren:
Eingestrahlte Lichtstrahlung wird mit einer wohl definierten Richtung nicht senkrecht auf die Gehäusewand 112, sondern unter einem beliebigen schrägen Winkel auf die Gehäusewand 112 gestrahlt. An der Grenzfläche zwischen der Gehäusewand 112 und dem Dialysat oder dem Blut sowie an allen weiteren Grenzflächen im Innenvolumenbereich 120 der Dialysatorvorrichtung 100 findet Lichtbrechung statt. Wie dem Fachmann bekannt ist, hängt diese vom Brechungsindex der an den Grenzflächen vorhandenen Stoffen ab. Aufgrund ihrer Konzentration und ihrer starken optischen Wechselwirkungen haben die optischen Eigenschaften des Dialysats und des Bluts den größten Effekt auf die Lichtbrechung. Die Messung von. Lichtbrechung kann somit zur Analyse von physikalischen Eigenschaften des Dialysats bzw. des Bluts herangezogen werden, z.B. zur Messung der Dichte.

Die Detektion der nach Lichtbrechung aus der Dialysatorvorrichtung 100 austretenden gebrochenen Lichtstrahlung kann ortsaufgelöst an einer von der Einstrahlstelle verschiedenen Stelle der Sensorvorrichtung 200 erfolgen. Der Ort der Detektion, z.B. durch selektive Auswahl aktiver Lichteintrittsöffnungen 256-x,y in der zweidimensionalen Anordnung 255 in Fig.3, und die Einfallsrichtung und -stelle des eingestrahlten Lichts können dabei aufeinander so abgestimmt werden, dass aus der Position des detektierten gebrochenen Lichts innerhalb der zweidimensionalen Anordnung 255 der Strahlungseintrittsöffnungen 256-x,y auf den bei der Lichtbrechung untergangenen Brechungswinkel zurückgeschlossen werden kann. Daraus kann auf den komplexen Brechungsindex und daraus wiederum auf die den Brechungsindex beeinflussenden physikalischen Eigenschaften der Flüssigkeiten im Dialysat bzw. im Blut, z.B. ihre Dichte, zurück geschlossen werden. Aus den physikalischen Eigenschaften lassen sich medizinisch relevante Parameter ableiten, wie etwa die Hämatokritkonzentration im Blut.

### 4. Transmissionsmessungen an der Dialysatorvorrichtung

Wird die Blickrichtung eines Detektors in der Sensorvorrichtung 200 an einer Stelle platziert und ausgerichtet, auf die die eingestrahlte einfallende Lichtstrahlung gerichtet ist, so lässt sich aus der Dämpfung bzw. aus der Abnahme der Lichtstrahlung beim Durchlaufen eines Laufwegs im Innenvolumenbereich 120, etwa im Blutbereich 130 oder im Diaysatbereich 170, ein Absorptionskoeffizient entlang der von der Lichtstrahlung durchlaufenen Laufstrecke bestimmen. Wird die Lichtstrahlung selektiv bezüglich der Absorptionswellenlänge gemessen, so kann durch Auswahl der Messwellenlänge eine stoffspezifische Absorption gemessen und auf eine Konzentration des entsprechenden Stoffes in dem Raumbereich entlang der Laufstrecke des Lichts zurück geschlossen werden. Derartige Transmissions- bzw. Absorptionsmessungen ermöglichen Rückschlüsse auf die stoffliche Zusammensetzung des Dialysats bzw. des Bluts.

### 5. Messung von Streustrahlung an der Dialysatorvorrichtung

Mit in den Beispielen 1 und 2 beschriebenen Messgeometrien kann auch die in der Dialysatorvorrichtung 100 gestreute Lichtstrahlung analysiert werden. Dazu kann an einer beliebigen Position an der Dialysatorvorrichtung 100, die sich möglichst weder in der Ausbreitungsrichtung des eingestrahlten noch in der Ausbreitungsrichtung des gebrochenen noch in der Ausbreitungsrichtung des reflektierten Lichts befindet, die Intensität des gestreuten Lichts bestimmt werden. Vorzugsweise wird dabei das gestreute Licht an mehreren Stellen an der Gehäuseoberfläche 112 der Dialysatorvorrichtung gemessen, was z.B. mit der in Fig. 3 gezeigten Ausführungsform einer Sensorvorrichtung 200 mit einer zweidimensional en Anordnung 255 von Lichteintrittsöffnungen 256-x,y möglich ist. Aus der Intensität des gestreuten Lichts lässt sich auf die Anwesenheit von Stoffen, z.B. bestimmten Molekülen, deren Abmessungen typischerweise bis zu 10-mal kleiner ist als die Wellenlänge des verwendeten Lichts, zurück schließen. Je größer die Konzentration derartiger Stoffe bzw. Strukturen, desto größer ist die Intensität der gestreuten Strahlung. Aus der räumlichen Verteilung der Intensität der gestreuten Strahlung in den unterschiedlichen Raumrichtungen lässt sich auch auf die Größe und die Form der Stoffe bzw. Strukturen (z.B. Moleküle) zurück schließen.

Ein Spezialfall von mit der Sensoreinrichtung 200 induzierbarer Lichtstreuung stellt die dynamische Lichtstreuung dar. Dabei wird eine stark monochromatische und stark gerichtete bzw. gebündelte Lichtquelle, wie etwa ein Laserstrahl, als Lichteinstrahlungseinrichtung 210 eingesetzt. Mittels einer zweidimensionalen Lichtdetektionsanordnung, wie etwa der zweidimensionalen Anordnung 255 der Lichteintrittsöffnungen 256-x,y in Fig. 3, kann die räumliche Verteilung des gestreuten Lichts gemessen werden. Die räumliche Verteilung wird unter anderem durch Interferenz des gestreuten eingestrahlten Lichts beeinflusst, wobei Licht auf unterschiedlichen Laufwegen (Streupfaden) von dem einen eingestrahlten Lichtstrahl einen gleichen Punkt der zweidimensionalen (flächigen) Detektoreinrichtung erreicht. Aus der gemessenen zeitlichen Veränderung des mit der flächigen Detektoreinrichtung gemessenen Interferenzmusters lässt sich auf die Bewegung der streuenden Zentren zurück schließen. Sind die analysierten Streuzentren beispielsweise rote Blutkörperchen, so kann auf deren Geschwindigkeit zurück geschlossen werden, insbesondere auf die Geschwindigkeit ihrer (ungerichteten) Brownschen Bewegung, und daraus auf die Koagulationsneigung des Bluts in Kapillaren der Membran der Membranfiltereinrichtung 190 und darauf basierend können gegebenenfalls Änderungen von Parametern der Dialysetherapie eingeleitet werden.

### 6. Elektromagnetische Identifizierung einer in der Dialysetherapie verwendeten Dialysator- bzw. Membranfiltereinrichtung

Elektromagnetische Strahlung kann auch eingesetzt werden, um die in einer Dialysetherapie an einem Patienten 10 verwendete Dialysatorvorrichtung 100 bzw. Membranfiltereinrichtung 190 automatisiert zu identifizieren. So kann die Dialysemaschine 300 Informationen über die in der Dialysetherapie eines Patienten 10 verwendete (z.B. mittels der Gehäusehalter 180, 182 gehaltene) und als Einmalartikel ausgebildete Dialysatorvorrichtung 100 oder Membranfiltereinrichtung 190 ermitteln, beispielsweise den Typ bzw. eine Seriennummer oder eine sonstige spezielle Ausgestaltung derselben, ohne dass eine die Therapie durchführende medizinische Fachkraft in möglicherweise fehlerbehafteter Weise Informationen bezüglich der Identifizierung der in der Dialysetherapie verwendeten Dialysatorvorrichtung 100 bzw. Membranfiltereinrichtung 190, z.B. mittels der Eingabe-/Ausgabeeinrichtung 310 der Dialysemaschine 300, einzugeben braucht.

In einer Ausführungsform ist zu diesem Zweck in der Sensorvorrichtung 200 z.B. ein Barcodescanner, ein Detektor oder eine Kamera (zum Erkennen eines Codes, z.B. einer Datamatrix bzw. eines 20-Codes oder eines Fluoreszenzcodes) integriert, und die Sensorvorrichtung 200 wird so auf der Dialysatorvorrichtung 100 platziert, dass der Barcode-Scanner bzw. der Detektor oder die Kamera einen auf der als Einmalartikel ausgebildeten Dialysatorvorrichtung 100 oder Membranfiltervorrichtung 190 angebrachten oder aufgedruckten Code, z.B. einen Barcode bzw. Datamatrix bzw. 20- oder 30-Code oder einen Fluoreszenzcode lesen kann. Mit optischen Detektoren kann auch eine Farbcode-Identifizierung, etwa von an dem Einmalartikel angebrachten oder aufgedruckten Farbcode oder über eine farbige Ausgestaltung des Dialysatorgehäuses 110 oder über eine spektrometrische bzw. eine true-colour

Lichtdetektoreinrichtung, und damit eine Identifizierung der in der Dialysetherapie verwendeten Dialysatorvorrichtung 100 oder Membranfiltereinrichtung 190 realisiert werden.

Alternativ zu einer auf optischer Detektion beruhenden Identifizierung durch die Sensoreinrichtung 200 kann eine auf der Messung von RF (Englisch: radio frequency) Strahlung basierte Kommunikation mit einem an der Dialysatorvorrichtung 100 oder an der Komponente wie etwa der Membranfiltereinrichtung 190 angebrachten RFID-Chip aufgenommen werden, und in einer dem Fachmann bekannten Weise von dem RFID-Chip gespeicherte, charakteristische Identifizierungsinformation abgerufen werden. Ein Vorteil der Identifizierung mittels RF-Strahlung ist, dass der in der Sensorvorrichtung 200 integrierte RF- Detektor sehr kurzreichweitig, z.B. induktiv, ausgelegt werden kann und auf diese Weise mit hoher Sicherheit nur der RFID-Chip derjenigen Dialysatorvorrichtung 100 gelesen wird, an der die Sensorvorrichtung 200 aktuell angebracht wird.

Die hierin beschriebene bzw. die in den beigefügten Patentansprüchen beanspruchten Ausführungsformen der Sensorvorrichtung 200 gemäß dem zweiten Aspekt der Erfindung und der Dialysatorvorrichtung 100 gemäß dem dritten Aspekt der Erfindung ermöglichen in einer für einen Fachmann offensichtlichen Weise u.a. folgende Vorteile zu erzielen:
1) Messungen von Informationen bezüglich eines Zustands, z.B. einer Dialysance, oder einer Stoffkonzentration, oder bezüglich einer Identifizierung einer als Einmalartikel ausgebildeten Dialysatorvorrichtung 100 oder Membranfiltereinrichtung 190 während des Betriebs in einer Dialysebehandlung eines Patienten 10.
2) Messungen mit vergleichsweise hoher zeitlicher Auflösung, z.B. in Zeitintervallen von einigen Sekunden bis Minuten, die während einer laufenden Dialysebehandlung therapeutische Eingriffe zur Optimierung der Behandlung ermöglichen.
3) Messungen, insbesondere berührungslos durchgeführt, von Information bezüglich des Zustands der Dialysatorvorrichtung 100 oder der Membranfilterienrichtung 190 direkt an der Dialysatorvorrichtung 100.
4) Die Sensorvorrichtung 200 ist wieder verwendbar und damit kosteneffizient im Hinblick darauf, dass die Dialysatorvorrichtung 100 oder zumindest die darin angeordnete Membranfiltereinrichtung 190 ein Einmalartikel ist. Dieser Vorteil ist solange valide, bis zukünftig eine etwaige in der Dialysatorvorrichtung 100 integrierte Sensorik kostengünstiger sein wird.

### Bezugszeichenliste:

- 10: Patient
- 20: Blutkreislauf
- 22: arterieller Zugang
- 24: arterielles Schlauchsystem
- 26: arterielle Blutpumpe
- 28: Bluteinlass
- 32: Blutauslass
- 34: venöses Schlauchsystem
- 36: venöser Zugang
- 40: Dialysatkreislauf
- 42: Wasseraufbereitung
- 44: Bikarbonat-Konzentrat
- 46: Bikarbonat-Pumpe
- 48: Bikarbonat-Zufluss
- 54: Säure-Konzentrat
- 56: Säure-Pumpe
- 58: Säure-Zufluss
- 60: Bilanzierungseinrichtung - Dialysatzugang
- 62, 62': Dialysatzufuhr-Zufluss
- 64: Flusspumpe-Dialysatzugang
- 66: Bypass-Ventil
- 68: Bypass-Leitung
- 72: Ventil Dialysator Eingang
- 74: Dialysateinlass
- 76: Dialysatauslass
- 78: Ventil Dialysator Ausgang
- 80: Bilanzierungseinrichtung - Dialysatausfluss
- 82, 82': Dialysatausfluss
- 84: Flusspumpe-Dialysatausfluss
- 86: Dialysatabfuhr
- 100: Dialysatorvorrichtung
- 102: Längsachse
- 104: Umfang
- 110: Gehäuse
- 111, 111': erster Koppelbereich
- 112: Gehäusewand
- 114: erster Fensterbereich
- 116: zweiter Fensterbereich
- 118: dritter Fensterbereich
- 120: Innenvolumenbereich
- 130: Blutbereich
- 170: Dialysatbereich
- 180: erster Gehäusehalter
- 182: zweiter Gehäusehalter
- 190: Membranfiltereinrichtung
- 192: Identifizierungseinrichtung
- 200: Sensorvorrichtung
- 210,210': Clipmanschette
- 211,211': zweiter Koppelbereich
- 210: Signalempfangseinrichtung
- 212: Identifizierungsleseeinrichtung
- 220: Strahlungsmesseinrichtung
- 222: erster Strahlungsdetektor
- 224: zweiter Strahlungsdetektor
- 230: Strahlungseintrittsbereich
- 231: 1-dimensionale Anordnung
- 232-x: Strahlungseintrittsbereich
- 233: 1-dimensionale Anordnung
- 234-y: Strahlungseintrittsbereich
- 235: 2-dimensionale Anordnung
- 236-x,y: Strahlungseintrittsbereich
- 240: Strahlungsaussendeeinrichtung
- 242: erste Strahlungsquelle
- 244: zweite Strahlungsquelle
- 250: Strahlungsaustrittsbereich
- 251: 1-dimensionale Anordnung
- 252-x: Strahlungsaustrittsbereich
- 253: 1-dimensionale Anordnung
- 254-y: Strahlungsaustrittsbereich
- 255: 2-dimensionale Anordnung
- 256-x,y: Strahlungsaustrittsbereich
- 300: Dialysemaschine
- 302: Maschinengehäuse
- 304,304': Gehäuserollen
- 310: Eingabe-/Ausgabeeinrichtung
- 320: Steuereinheit
- 330: Datenverarbeitungs- bzw. Auswerteeinheit
- 340: Speichereinheit
- 360: Datenbussystem
- 362: Eingabe-/Ausgabe-Datenleitu ng
- 364: Steuerleitung
- 366: Betriebsdatenleitung
- 368: Datenspeicherleitung
- 370: Dialysatorvorrichtungssignalleitung
- 372: Sensorvorrichtungssignalleitung

## Patentansprüche

1. System zur Erfassung einer Eigenschaft oder eines Zustands einer Dialysatorvorrichtung (100) oder einer Komponente derselben vor oder während des Betriebs der Dialysatorvorrichtung (100), wobei das System folgendes umfasst:
die Dialysatorvorrichtung (100) mit einem einen Innenvolumenbereich (120) umschließenden Gehäuse (110) und einer in dem Innenvolumenbereich (120) angeordneten Membranfiltereinrichtung (190), wobei das Gehäuse (110) für ein Signal, wie etwa ein Strahlungssignal, zumindest bereichsweise durchlässig ist, und
eine Sensorvorrichtung (200), die dafür angepasst ist, um direkt mit dem Gehäuse (110) der Dialysatorvorrichtung (100) lösbar verbunden zu werden und die eine Signalempfangseinrichtung (210, 212, 220, 222, 224) aufweist, die dazu ausgebildet ist, mindestens ein Strahlungssignal zumindest aus dem Innenvolumenbereich (120) des Gehäuses (110) zu empfangen, wobei das Signal charakteristisch für die Dialysatorvorrichtung oder deren Zustand oder für die Blutbehandlung ist,
**dadurch gekennzeichnet, dass**
das Gehäuse (110) der Dialysatorvorrichtung (100) an seiner Außenseite einen ersten Koppelbereich (111, 111') aufweist,
die Sensorvorrichtung (200) als eine Clipeinrichtung (220) mit mindestens einem oder mehreren flexiblen Cliparmen bzw. Clipmanschetten (210, 210') ausgebildet ist, die das Gehäuse (110) der Dialysatorvorrichtung (100) entlang seiner Längsrichtung in einem Winkelbereich von mehr als 180° umgreift und einen zweiten Koppelbereich (211,211') aufweist, der zu dem ersten Koppelbereich (111,111') an dem Gehäuse (110) des Dialysators (100) formschlüssig ausgebildet ist, wobei
der erste und der zweiten Koppelbereich (111, 111', 211, 211') dazu angeordnet sind, derart zusammenzuwirken, dass die Sensorvorrichtung (200) an einer vorbestimmten Position entlang der Längsrichtung des Gehäuses (110) des Dialysators (100) lösbar an diesem anbringbar ist.

2. System nach Anspruch 1, das dazu ausgebildet ist, das empfangene Signal einer Auswerteeinrichtung (330) bereitzustellen, die auf der Grundlage des empfangenen Signals Daten erzeugt, die zum Steuern von Betriebsparametern einer Dialysemaschine (300) verwendet werden können.

3. System nach Anspruch 1 oder 2, bei dem die Signalempfangseinrichtung (210, 220, 222, 224) dazu ausgebildet ist, Signale einer Strahlung zu empfangen oder zu detektieren, die ausgewählt ist aus einer Gruppe, die folgendes umfasst:
a) elektromagnetische Strahlung des gesamten elektromagnetischen Spektrums, optional mit einer Wellenlänge im optischen Bereich wie etwa im Bereich ferninfraroten, infraroten (IR), nahinfraroten, sichtbaren und ultravioletten (UV) Lichts,
b) elektromagnetische Strahlung mit einer Wellenlänge bzw. dieser entsprechenden Frequenz im Mikrowellenbereich, im Terahertzbereich, d.h. im Submillimeterstrahlungsbereich, oder im Bereich von Radiowellen, und
c) Ultraschall-Strahlung.

4. System nach Anspruch 1 oder 2, bei dem die Signalempfangseinrichtung (210) einen Empfänger für ein elektrisches Signal umfasst, wobei das elektrische Signal indikativ für eine zu messende Kapazität und/oder eine zu messende Induktivität ist, die charakteristisch für den Zustand bzw. die Identifizierung der Dialysatorvorrichtung (100) und/oder der Membranfiltereinrichtung (190) ist.

5. System nach Anspruch 3, bei dem die Sensorvorrichtung (200) eine Strahlungsaussendeeinrichtung (240) umfasst, die dazu ausgebildet ist, eine vorbestimmte Strahlung in den Innenvolumenbereich (120) des Gehäuses (110) der Dialysatorvorrichtung (100) auszusenden, und dass
die Signalempfangseinrichtung (210, 220, 222, 224) dazu ausgebildet ist, einen oder mehrere Parameter wie etwa die Intensität, Phase und/oder das Zeitverhalten von Strahlung, die von der Strahlungsaussendeeinrichtung (240) ausgesendet worden ist, zu messen, wobei die gemessene Strahlung das Ergebnis einer für den Zustand der Dialysatorvorrichtung (200) charakteristischen Wechselwirkung sein kann, die im Innenvolumenbereich (120) des Gehäuses (110) zwischen der Strahlung und einem oder mehreren der folgenden stattgefunden hat:
- dem Dialysat bzw. dem Blut,
- einem in dem Dialysat und/oder in dem Blut enthaltenen Stoff,
- der Membranfiltereinrichtung (190) und
- einem in bzw. an der Membranfiltereinrichtung (190) festgehaltenen Stoff, der aus dem Blut bzw. dem Dialysat entstammt,
wobei zumindest ein Teil der wechselwirkenden Strahlung aus dem Innenvolumenbereich (120) zu der Signalempfangseinrichtung (210, 220, 222, 224) gelangt ist, und
wobei die Wechselwirkung durch von der Strahlungsaussendeeinrichtung (240) in den Innenvolumenbereich (120) des Gehäuses (110) ausgesendeten Strahlung hergerufen worden ist.

6. System nach Anspruch 5, bei dem eine optische Wechselwirkung hervorgerufen wird, die ausgewählt ist aus einer Gruppe, die folgendes umfasst:
- Reflektion von von der Strahlungsaussendeeinrichtung (240) ausgesendeter optischer Strahlung an einer Grenzfläche zwischen der Gehäusewand (112) und dem Dialysat oder dem Blut,
- Reflektion von von der Strahlungsaussendeeinrichtung (240) ausgesendeter optischer Strahlung an einer Grenzfläche zwischen der Membranfiltereinrichtung (190) und dem Dialysat oder dem Blut,
- Transmission von von der Strahlungsaussendeeinrichtung (240) ausgesendeter optischer Strahlung mit einer Messwellenlänge, die von einem in dem Dialysat und/oder in dem Blut enthaltenen Stoff absorbiert werden kann und die auf ihrem Weg zu der Strahlungsempfangseinrichtung (220) einen Strahlungslaufweg durch das Dialysat und/oder durch das Blut zurückgelegt hat,
- Emission von Lumineszenz- oder Fluoreszenzstrahlung durch einen in dem Dialysat und/oder in dem Blut enthaltenen Stoff, wobei eine Lumineszenz- oder Fluoreszenzreaktion in diesem Stoff durch von der Strahlungsaussendeeinrichtung (240) ausgesendete optischer Strahlung hervorgerufen worden ist,
- Brechung von von der Strahlungsaussendeeinrichtung (240) ausgesendeter optischer Strahlung an einer Grenzfläche zwischen der Gehäusewand (112) und dem Dialysat oder dem Blut, wobei die ausgesendete Strahlung unter einem zwischen 0° und 180° oder 0° und 90° liegenden Einfallswinkel auf der Grenzfläche auftrifft,
- Streuung von von der Strahlungsaussendeeinrichtung (240) ausgesendeter optischer Strahlung an einem in dem Dialysat oder in dem Blut enthaltenen Stoff, einschließlich dynamischer Streuung von monochromatischer Laserstrahlung, und
- Wechselwirkung von von der Strahlungsaussendeeinrichtung (240) ausgesendeter optischer Strahlung mit einer Identifizierungseinrichtung (192), die an der Dialysatorvorrichtung (100) oder an der Membranfiltereinrichtung (190) angebracht ist, die ein für die Identifizierung der Dialysatorvorrichtung (100) oder der Membranfiltereinrichtung (190) charakteristisches Identifizierungsmerkmal aufweist.

7. System nach Anspruch 5 oder 6, bei dem die Strahlungsaussendeeinrichtung (240) mindestens eines oder mehrere der folgenden Merkmale umfasst:
- eine Lichtquelle, die Licht in einem schmalbandigen Spektralbereich aussendet,
- eine Lichtleitfaser, die aus einer Licht in einem schmalbandigen Spektralbereich aussendenden Lichtquelle ausgesendetes Licht führt und einen Auskopplungsabschnitt aufweist, aus dem das Licht austritt,
- eine Lichtquelle, die Licht in einem breitbandigen Spektralbereich aussendet,
- eine Lichtleitfaser, die aus einer Licht in einem breitbandigen Spektralbereich aussendenden Lichtquelle ausgesendetes Licht führt und einen Auskopplungsabschnitt aufweist, aus dem das Licht austritt.

8. System nach Anspruch 7, bei dem die Strahlungsaussendeeinrichtung (240) eine 1-dimensionale Anordnung (231, 233) oder eine 2-dimensionale Anordnung (236) von mehreren Strahlungsaustrittsbereichen (250) umfasst, wobei die Anordnung (231, 233, 235) im verbundenen Zustand der Sensorvorrichtung (200) und der Dialysatorvorrichtung (100) quer, schräg oder im Wesentlichen parallel zu einer Strömungsrichtung des Dialysats in dem Dialysatbereich (170) oder quer, schräg oder parallel zu der Strömungsrichtung des Bluts in dem Blutbereich (130) oder quer, schräg oder parallel zu einer Längsachse (102) oder entlang des Umfangs der Dialysatorvorrichtung (100) ausgerichtet ist.

9. System nach Anspruch 7 oder 8, bei dem die Sensorvorrichtung (200) eine Strahlungsmesseinrichtung (220) mit einer Messoptikeinrichtung, die eine Fokaltiefe und eine Fokalrichtung aufweist, und mit einem Lichtdetektor, der eine lichtsensitive Detektorfläche aufweist, umfasst, wobei die Messoptikeinrichtung dazu ausgebildet ist, einen Raumbereich aus dem Innenvolumenbereich (120) des Gehäuses (110) der Dialysatorvorrichtung (100) auf die Detektorfläche des Lichtdetektors abzubilden, wobei der abgebildete Raumbereich durch die Fokaltiefe und die Fokalrichtung definiert ist, und wobei die Messoptikeinrichtung so ausgebildet ist, dass ihre Fokaltiefe und Fokalrichtung vor der Inbetriebnahme oder während des Betriebs so verstellbar oder wählbar ist, dass der abgebildete Raumbereich im Bereich des Innenvolumenbereichs (120) wählbar ist.

10. System nach einem der vorhergehenden Ansprüche, bei dem die Sensorvorrichtung (200) eine Strahlungsmesseinrichtung (220) mit mindestens einem Lichtdetektor und einer Wellenlängenauswahleinrichtung umfasst, wobei die Wellenlängenauswahleinrichtung dazu ausgebildet ist, einen schmalbandigen Wellenlängenbereich, der eine Nachweiswellenlänge umfasst, aus dem breitbandigeren Wellenlängenbereich der von der Strahlungsaussendeeinrichtung (24) ausgesendeten Strahlung, z.B. Licht, einstrahlungsseitig oder empfangsseitig so auszuwählen, dass eine optische Wechselwirkung mit einem ausgewählten Stoff, der sich im Betrieb an oder in der Dialysatorvorrichtung oder in dem Innenvolumenbereich (120) des Gehäuses (110) befindet, erfolgt.

11. System nach einem der Ansprüche 1 bis 10, bei dem die Sensorvorrichtung (200) dazu ausgebildet ist, mindestens einen der folgenden Parameter zu erfassen:
im Blutbereich (30, 130) des Innenvolumenbereichs (120), Messen eines oder mehrerer für die Konzentration eines oder mehrerer Stoffe indikativen Parameters die von dem Stoff absorbiert werden,
im Dialysatbereich (70, 170) des Innenvolumenbereichs (120), Messen eines für die Konzentration eines oder mehrerer Stoffe indikativen Parameters,
im Blutbereich (30, 130), Messen eines für eine physikalische Eigenschaft des Bluts indikativen Parameters.

12. Sensorvorrichtung (200), die dafür ausgebildet ist, um direkt mit einem Gehäuse (110) einer Dialysatorvorrichtung (100) lösbar verbunden zu werden und die mindestens einen Sensor zur Erfassung eines aus dem Inneren des Gehäuses stammenden Signals aufweist, wobei durch die Sensorvorrichtung in einem mit der Dialysatorvorrichtung (100) verbundenen Zustand ein System gemäß einem der Ansprüche 1 bis 11 ausbildbar ist,
**dadurch gekennzeichnet, dass**
die Sensorvorrichtung (200) als eine Clipeinrichtung (220) mit mindestens einem oder mehreren flexiblen Cliparmen bzw. Clipmanschetten (210, 210') ausgebildet ist, die dazu angeordnet ist, das Gehäuse (110) der Dialysatorvorrichtung (100) entlang seiner Längsrichtung in einem Winkelbereich von mehr als 180° zu umgreifen, und die einen zweiten Koppelbereich (211, 211') aufweist, der zu einem ersten Koppelbereich (111, 111') an dem Gehäuse (110) des Dialysators (100) formschlüssig ausgebildet ist, wobei
der erste und der zweiten Koppelbereich (111, 111', 211, 211') dazu angeordnet sind, derart zusammenzuwirken, dass die Sensorvorrichtung (200) an einer vorbestimmten Position entlang der Längsrichtung des Gehäuses (110) des Dialysators (100) lösbar an diesem anbringbar ist..

13. Sensorvorrichtung (200) nach Anspruch 12, die mindestens einen Strahlungssender zur Aussendung von Strahlung auf die Oberfläche und/oder in das Innere der Dialysatorvorrichtung aufweist, wobei die Sensorvorrichtung wieder verwendbar ausgebildet ist.

14. Sensorvorrichtung (200) nach Anspruch 12 oder 13, bei der für die an dem Gehäuse (110) lösbar, z.B. formschlüssig befestigbare Ausbildung der Sensorvorrichtung (200) mindestens einer der folgenden Mechanismen vorgesehen ist:
die Sensorvorrichtung (200) umfasst einen Grundkörper und eine Clipeinrichtung mit mindestens einem oder zwei an dem Grundkörper befestigten elastisch-flexiblen Cliparmen oder an dem Grundkörper elastisch-flexibel angelenkten Cliparmen, der oder die dazu ausgebildet sind, das Gehäuse (110) zumindest teilweise zu umgreifen oder mit diesem eine feste lösbare Verbindung einzugehen oder aufgrund ihrer elastisch-flexiblen Ausgestaltung oder Anlenkung das Gehäuse (110) clipartig zu umklammern,
die Sensorvorrichtung (100) umfasst einen Grundkörper und mindestens einen Arm oder ein Paar von an dem Grundkörper angelenkten oder mit dem Grundkörper elastisch-flexibel verbundenen Armen bzw. Manschetten, die jeweils einen distalen Endbereich mit einem dort vorgesehenen ersten Glied einer Haken- oder Eingreifeinrichtung aufweisen, wobei der oder die Arme bzw. Manschetten dazu ausgebildet sind, das Gehäuse (110) in einem Winkelbereich von mehr als 180° zu umgreifen, und eine elastisch spannbare Spanneinrichtung mit einem oder zwei gegenüberliegenden Endabschnitten, an denen jeweils ein zum lösbaren Eingreifen mit einem ersten Glied der Haken- oder Eingreifeinrichtung ausgebildetes zweites Glied einer Haken- oder Eingreifeinrichtung vorgesehen sein kann, wobei in einem elastisch gespannten Zustand der Spanneinrichtung ein jeweiliges zweites Glied am Endabschnitt der Spanneinrichtung mit einem ersten Glied an den Endbereichen der Arme bzw. Manschetten der Sensorvorrichtung (200) eingreifen kann, und
die Sensorvorrichtung (200) ist dazu ausgebildet, in der Gehäusewand (112) der Dialysatorvorrichtung (100) lösbar integriert zu werden.

15. Dialysatorvorrichtung (100) mit einem einen Innenvolumenbereich (120) umschließenden Gehäuse (110) und einer in dem Innenvolumenbereich (120) angeordneten Membranfiltereinrichtung (190), wobei das Gehäuse (110) für ein Signal, wie etwa ein Strahlungssignal, zumindest bereichsweise durchlässig ist, und die Dialysatorvorrichtung (100) so ausgebildet ist, dass mit ihr eine Sensorvorrichtung nach einem der Ansprüche 12 bis 14 lösbar operativ verbindbar und in einem verbundenen Zustand ein System gemäß einem der Ansprüche 1 bis 11 ausbildbar ist,
**dadurch gekennzeichnet, dass**
das Gehäuse (110) der Dialysatorvorrichtung (100) an seiner Außenseite einen ersten Koppelbereich (111, 111') aufweist, der zu einem zweiten Koppelbereich (211, 211') an einer das Gehäuse (110) der Dialysatorvorrichtung (100) entlang seiner Längsrichtung in einem Winkelbereich von mehr als 180° umgreifenden und mit mindestens einem oder mehreren flexiblen Cliparmen bzw. Clipmanschetten (210, 210') ausgebildeten Clipeinrichtung (220) der Sensorvorrichtung (200) formschlüssig ausgebildet ist, wobei
der erste und der zweiten Koppelbereich (111, 111', 211, 211') dazu angeordnet sind, derart zusammenzuwirken, dass die Sensorvorrichtung (200) an einer vorbestimmten Position entlang der Längsrichtung des Gehäuses (110) des Dialysators (100) lösbar an diesem anbringbar ist.

16. Dialysatorvorrichtung (100) nach Anspruch 15, mit mindestens einem, wahlweise mehreren oder allen der folgenden Merkmale:
das Gehäuse (100) umfasst eine Gehäusewand (112) mit mindestens einem für ein Signal durchlässigen Fensterbereich (114, 116, 118);
die Membranfiltereinrichtung (190) kann in das Gehäuse (100) herausnehmbar eingebracht werden, ein Innengehäuse kann in dem Gehäuse (110) lösbar fixiert werden und umschließt die Membranfiltereinrichtung (190),
das Innengehäuse kann in das Gehäuse (100) herausnehmbar eingebracht werden,
zumindest die Membranfiltereinrichtung (190) ist als Einmalartikel ausgebildet,
das Innengehäuse und die Membranfiltereinrichtung (190), oder die Dialysatorvorrichtung (100) insgesamt, sind als Einmalartikel ausgebildet,
der Innenvolumenbereich (120) umfasst einen im Betrieb von einem Dialysat durchströmbaren Dialysatbereich (170) und einen im Betrieb von Blut durchströmbaren Blutbereich (130), wobei der Dialysatbereich (170) von dem Blutbereich (130) durch die Membranfiltereinrichtung (190) räumlich getrennt sind,
die Membranfiltereinrichtung (190) ist dazu ausgebildet, im Betrieb einen durch Konzentrationsgradienten von in dem Blut bzw. in dem Dialysat gelösten Stoffen getriebenen Stoffaustausch zwischen dem Blut und dem Dialysat zu ermöglichen,
die Dialysatorvorrichtung ist als intelligenter Dialysator ausgebildet und / oder weist Schnittstellen zu einer externen Steuereinrichtung auf.

17. Dialysemaschine (300) zum Durchführen einer Dialysebehandlung an einem Patienten, mit einer Dialysatorvorrichtung (100) nach einem der Ansprüche 15 oder 16 und einer Sensorvorrichtung (200) nach einem der Ansprüche 12 bis 14, die dazu vorgesehen sind, ein System gemäß einem der Ansprüche 1 bis 11 auszubilden oder zu einem derartigen System lösbar operativ verbunden zu werden.

18. Dialysemaschine (300) nach Anspruch 17, bei der die Dialysatorvorrichtung (100) zumindest einen Gehäusehalter (180, 182) umfasst, der separat ausgebildet oder mit der Dialysemaschine fest verbunden ist und dazu ausgebildet ist, mit der Dialysemaschine (300) zumindest im Betrieb mechanisch verbunden zu sein und die Dialysatorvorrichtung (100) lösbar zu fixieren.

19. Dialysemaschine (300) nach Anspruch 18, bei der die Sensorvorrichtung (200) in dem Gehäusehalter (180, 182) integriert ist.

## Claims

1. A system for detecting a property or a state of a dialyzer apparatus (100) or a component thereof before or during the operation of the dialyzer apparatus (100), with the system comprising the following:
the dialyzer apparatus (100) having a housing (110) encompassing an internal volume portion (120) and a membrane filter device (190) disposed in the internal volume portion (120), where the housing (110) is permeable at least in regions to a signal, such as a radiation signal, and
a sensor device (200) that is adapted to be connected directly to the housing (110) of the dialyzer apparatus (100) in a detachable fashion and that comprises a signal receiving unit (210, 212, 220, 222, 224) that is designed to receive at least one radiation signal at least from the internal volume portion (120) of the housing (110), with the signal being characteristic for the dialyzer apparatus or the state thereof or for the blood treatment,
**characterized in that**
the housing (110) of the dialyzer apparatus (100) comprises a first coupling region (111, 111') at its exterior side,
the sensor device (200) is formed as a clip unit (220) having at least one or more flexible clip arms or clip cuffs (210, 210'), which encompasses the housing (110) of the dialyzer apparatus (100) in an angular range of more than 180° along the longitudinal direction of the housing (110) and comprises a second coupling region (211,211') that is formed to engage in a positive fit with the first coupling region (111, 111') at the housing (110) of the dialyzer apparatus (100), wherein
the first and second coupling regions (111, 111', 211, 211') are arranged to interact such that the sensor device (200) is mountable in a detachable fashion at the housing (110) of the dialyzer apparatus (100) at a predetermined position along its longitudinal direction.

2. The system according to Claim 1 that is designed to provide the received signal of an analysis unit (330) that generates data on the basis of the received signal that may be used for controlling the operating parameters of a dialysis machine (300).

3. The system according to Claim 1 or 2, wherein the signal receiving unit (210, 220, 222, 224) is designed to receive or detect signals of a radiation selected from a group that comprises the following:
a) electromagnetic radiation of the entire electromagnetic spectrum, optionally with a wavelength in the optical range, such as in the region of far infrared, infrared (IR), near infrared, visible, and ultraviolet (UV) light,
b) electromagnetic radiation having a wavelength or a frequency corresponding thereto in the microwave range, in the terahertz range, i.e., in the submillimeter radiation range, or in the range of radio waves, and
c) ultrasound radiation.

4. The system according to Claim 1 or 2, wherein the signal receiving unit (210) comprises a receiver for an electrical signal, with the electrical signal being indicative of a capacity to be measured and/or an inductivity to be measured that is characteristic of the state and/or the identification of the dialyzer apparatus (100) and/or the membrane filter unit (190).

5. The system according to Claim 3, with the sensor device (200) comprising a radiation transmitting unit (240) that is designed to transmit a predetermined radiation into the internal volume portion (120) of the housing (110) of the dialyzer apparatus (100), and with
the signal receiving unit (210, 220, 222, 224) being designed to measure one or more parameters such as the intensity, phase, and/or the behavior over time of radiation emitted by the radiation transmitting unit (240), where the measured radiation may optionally be the result of an interaction characteristic for the state of the dialyzer apparatus (200) that has occurred in the internal volume portion (120) of the housing (110) between the radiation and one or more of the following:
- the dialysate and/or the blood,
- a material contained in the dialysate and/or in the blood,
- the membrane filter device (190), and
- a material held in and/or on the membrane filter device (190) originating from the blood and/or the dialysate,
with at least a portion of the interacting radiation having arrived from the internal volume portion (120) to the signal receiving unit (210, 220, 222, 224), and
with the interaction being incurred by radiation transmitted from the radiation transmitting unit (240) into the internal volume portion (120) of the housing (110).

6. The system according to Claim 5, wherein an optical interaction is incurred that has been selected from a group that comprises the following:
- reflection of optical radiation emitted by the radiation transmitting unit (240) on a boundary surface between the housing wall (112) and the dialysate or the blood,
- reflection of optical radiation emitted by the radiation transmitting unit (240) on a boundary surface between the membrane filter device (190) and the dialysate or the blood,
- transmission of optical radiation emitted by the radiation transmitting unit (240) having a measuring wavelength that can be absorbed by a substance contained in the dialysate and/or in the blood and, on its way to the radiation receiving unit (220), has followed a beam path through the dialysate and/or through the blood,
- emission of luminescent or fluorescent radiation by a material contained in the dialysate and/or in the blood, with a luminescent or fluorescent reaction in said material being triggered by the optical radiation emitted by the radiation transmitting unit (240),
- refraction of the optical radiation emitted by the radiation transmitting unit (240) on a boundary surface between the housing wall (112) and the dialysate or the blood, with the transmitted radiation striking the boundary surface at an angle of incidence between 0° and 180° or between 0° and 90°,
- scattering of optical radiation emitted by the radiation transmitting unit (240) on a substance contained in the dialysate or in the blood, including dynamic scattering of monochromatic laser radiation, and
- interaction of optical radiation emitted by the radiation transmitting unit (240) with an identification unit (192) attached to the dialyzer apparatus (100) or the membrane filter device (190) that comprises an identifying feature characteristic for the identification of the dialyzer apparatus (100) or the membrane filter device (190).

7. The system according to Claim 5 or 6, in which the radiation transmitting unit (240) comprises at least one or more of the following features:
- a light source that transmits light in a narrow-band spectral range,
- a light-conducting fiber that guides light transmitted by a light source emitting light in a narrow-band spectral range and comprises a decoupling segment from which the light exits,
- a light source that transmits light in a wide-band spectral range,
- a light-conducting fiber that guides light transmitted by a light source emitting light in a wide-band spectral range and comprises a decoupling segment from which the light exits.

8. The system according to Claim 7, in which the radiation transmitting unit (240) comprises a one-dimensional arrangement (231, 233) or a two-dimensional arrangement (236) of a plurality of light exit regions (250), in which the arrangement (231, 233, 235) in the connected state of the sensor device (200) and the dialyzer apparatus (100) is oriented transversely, obliquely, or parallel to a flow direction of the dialysate in the dialysate region (170) or transversely or obliquely or parallel to the flow direction of the blood in the blood region (130) or transversely, obliquely, or essentially parallel to a longitudinal axis (102) or along the circumference of the dialyzer apparatus.

9. The system according to Claim 7 or 8, in which the sensor device (200) comprises a radiation measuring unit (220) having a measurement optic unit having a focal depth and a focal direction, and having a light detector comprising a light-sensitive detector surface, wherein the measurement optic unit is designed to map a spatial region from the internal volume portion (120) of the housing (110) of the dialyzer apparatus (100) on the detector surface of the light detector, with the mapped spatial region being defined by the focal depth and the focal direction and with the measurement optic unit being designed in such a way that its focal depth and focal direction may be adjusted or selected before startup or during operation in such a way that the spatial region to be mapped inside the internal volume portion (120) is selectable.

10. The system according to one of the preceding Claims, in which the sensor device (200) comprises a radiation measuring unit (220) having at least one light detector and a wavelength selection unit with the wavelength selection unit being designed to select a narrow-band wavelength range including a reference wavelength range from the wider-band wavelength range of the radiation emitted by the radiation transmitting unit (24), for example, light, on the transmitting side or on the receiving side in such a way that an optical interaction occurs with a selected material located during operation on or in the dialyzer apparatus or in the internal volume portion (120) of the housing (110).

11. The system according to one of Claims 1 to 10, wherein the sensor device (200) is designed to detect at least one of the following parameters:
in the blood region (30, 130) of the internal volume portion (120), the measurement of one or more parameters indicative of the concentration of one or more substances that are absorbed by the substance,
in the dialysate region (70, 170) of the internal volume portion (120), the measurement of a parameter indicative of the concentration of one or more substances
in the blood region (30, 130), measurement of a parameter indicative of a physical property of the blood.

12. A sensor device (200) that is designed to be connected directly in a removable fashion to a housing (110) of a dialyzer apparatus (100) and that comprises at least one sensor for detecting a signal originating from the interior of the housing, wherein the sensor device in its connected state to the dialyzer apparatus (100) is able to form a system according to one of Claims 1 to 11
**characterized in that**
the sensor device (200) is formed as a clip unit (220) having at least one or more flexible clip arms or clip cuffs (210, 210'), which is arranged to encompass the housing (110) of the dialyzer apparatus (100) in an angular range of more than 180° along the longitudinal direction of the housing (110) and comprises a second coupling region (211, 211') that is formed to engage in a positive fit with the first coupling region (111, 111') at the housing (110) of the dialyzer apparatus (100), wherein
the first and second coupling regions (111, 111', 211, 211') are arranged to interact such that the sensor device (200) is mountable in a detachable fashion at the housing (110) of the dialyzer apparatus (100) at a predetermined position along its longitudinal direction.

13. The sensor device (200) according to Claim 12 that comprises at least one radiation transmitter for transmitting radiation onto the surface and/or into the interior of the dialyzer apparatus, with the sensor device being optionally designed to be reusable.

14. The sensor device (200) according to Claim 12 or 13, wherein at least one of the following mechanisms is provided for the embodiment of the sensor device (200) to be attachable in a removable fashion, for example, with a positive fit, on the housing (110):
the sensor device (200) comprises a base body and a clip unit having at least one or two elastically flexible clip arms attached to the base body or elastically flexible clip arms articulated on the base body designed to encompass the housing (110) at least partially or to enter into a fixed, removable connection therewith or, due to its elastically flexible design or articulation, to encompass the housing (110) in a clip-like fashion,
the sensor device (100) comprises a base body and at least one arm or a pair of arms or cuffs articulated on the base body or connected to the base body in an elastically flexible fashion, each comprising a distal end region having a first member of a hook or engaging unit provided thereon, with the arm or the arms and/or cuffs being designed to encompass the housing (110) in an angular range of more than 180°, and an elastically tensible clamping unit having one or two end sections opposite one another, on each of which a second member of a hook or engaging unit is provided that is designed to detachably engage a first member of the hook or engaging unit, with each second member on the end section of the clamping unit being able to engage with a first member on the end regions of the arms and/or clamps of the sensor device (200), and
the sensor device (200) is designed to be integrated into the housing wall (112) of the dialyzer apparatus (100) in a removable fashion.

15. A dialyzer apparatus (100) having a housing (110) encompassing an internal volume portion (120) and a membrane filter device (190) disposed in the internal volume portion (120), with the housing (110) being permeable at least in regions to a signal, for example, a radiation signal, and with the dialyzer apparatus (100) being designed in such a way that a sensor device according to one of Claims 12 to 14 may be operatively connected thereto in a removable fashion and, in a connected state, is able to form a system according to one of Claims 1 to 11,
**characterized in that**
the housing (110) of the dialyzer apparatus (100) comprises a first coupling region (111, 111') at its exterior side, which is formed to engage in a positive fit with a second coupling region (211, 211') at a clip unit (220) of the sensor device (200), the clip unit (220) encompasses the housing (110) of the dialyzer apparatus (100) in an angular range of more than 180° along the longitudinal direction of the housing (110) and having at least one or more flexible clip arms or clip cuffs (210, 210', wherein
the first and second coupling regions (111, 111', 211, 211') are arranged to interact such that the sensor device (200) is mountable in a detachable fashion at the housing (110) of the dialyzer apparatus (100) at a predetermined position along its longitudinal direction.

16. The dialyzer apparatus (100) according to Claim 15, having at least one of the following features and optionally having more than one or all of the following features:
the housing (100) comprises a housing wall (112) having at least one window region (114, 116, 118) that is permeable to a signal;
the membrane filter device (190) may be placed in the housing (100) in a removable fashion; an interior housing may be removably fixed in the housing (110) and encompasses the membrane filter device (190),
the inner housing may be removably inserted in the housing (100),
at least the membrane filter device (190) is designed as a single-use article,
the inner housing and the membrane filter device (190), or the dialyzer apparatus (100) as a whole, are designed as single-use articles,
the internal volume portion (120) comprises a dialysate region (170) through which dialysate is able to flow during operation and a blood region (130) through which blood is able to flow during operation, with the dialysate region (170) being spatially separated from the blood region (130) by the membrane filter unit (190),
the membrane filter unit (190) is designed to allow a material exchange between the blood and the dialysate during operation driven by concentration gradients of materials dissolved in the blood and/or in the dialysate,
the dialyzer apparatus is designed as an intelligent dialyzer and/or comprises interfaces to an external control unit.

17. A dialysis machine (300) for conducting a dialysis treatment on a patient, having a dialyzer apparatus (100) according to one of Claims 15 or 16 and a sensor device (200) according to one of Claims 12 to 14 that are optionally provided to form a system according to one of Claims 1 to 11 or to be operatively connected to such a system in a removable fashion.

18. The dialysis machine (300) according to Claim 17, wherein the dialyzer apparatus (100) comprises at least one housing holder (180, 182) that is embodied separately or connected in a fixed fashion to the dialysis machine and that is designed to be mechanically connected to the dialysis machine (300) at least during operation and to fix the dialyzer apparatus (100) in a removable fashion.

19. The dialysis machine (300) according to Claim 18, wherein the sensor device (200) is integrated into the housing holder (180, 182).

## Revendications

1. Système de détection d'une propriété ou d'un état d'un dispositif dialyseur (100) ou d'un composant de celui-ci avant ou pendant le fonctionnement du dispositif dialyseur (100), le système comprenant ce qui suit :
le dispositif dialyseur (100), avec un boîtier (110) renfermant une zone de volume intérieur (120) et une installation de filtration à membrane (190) agencée dans la zone de volume intérieur (120), le boîtier (110) étant perméable au moins par endroits à un signal, tel qu'un signal de rayonnement, et
un dispositif capteur (200) qui est adapté pour être relié directement au boîtier (110) du dispositif dialyseur (100) de façon amovible et qui comprend un dispositif de réception de signaux (210, 212, 220, 222, 224) configuré pour recevoir au moins un signal de rayonnement provenant au moins de la zone de volume intérieur (120) du boîtier (110), le signal étant caractéristique du dispositif dialyseur ou de son état ou
du traitement du sang,
**caractérisé en ce que**
le boîtier (110) du dispositif dialyseur (100) comprend sur sa face extérieure une première zone de couplage (111, 111'),
le dispositif capteur (200) est configuré comme un dispositif de clipsage (220) avec au moins un ou plusieurs bras de clipsage ou manchons de clipsage flexibles (210, 210'), qui entoure le boîtier (110) du dispositif dialyseur (100) suivant sa direction longitudinale dans une plage angulaire de plus de 180° et comprend une deuxième zone de couplage (211, 211') qui est formée avec complémentarité de formes par rapport à la première zone de couplage (111, 111') sur le boîtier (110) du dialyseur (100),
les première et deuxième zones de couplage (111, 111', 211, 211') étant agencées pour coopérer de telle sorte que le dispositif capteur (200) peut être monté de façon amovible sur le boîtier (110) du dialyseur (100) en une position prédéterminée suivant la direction longitudinale de celui-ci.

2. Système selon la revendication 1, qui est configuré pour fournir le signal reçu d'un dispositif d'évaluation (330) qui, sur la base du signal reçu, génère des données pouvant être utilisées pour commander des paramètres de fonctionnement d'une machine de dialyse (300).

3. Système selon la revendication 1 ou 2, dans lequel le dispositif de réception de signaux (210, 220, 222, 224) est configuré pour recevoir ou détecter des signaux d'un rayonnement qui est sélectionné parmi un groupe comprenant ce qui suit :
a) rayonnement électromagnétique de l'ensemble du spectre électromagnétique, facultativement avec une longueur d'ondes dans le domaine optique tel que le domaine de la lumière infrarouge lointain, infrarouge (IR), proche infrarouge, visible et ultraviolette (UV),
b) rayonnement électromagnétique avec une longueur d'ondes, respectivement cette fréquence correspondante, dans le domaine des micro-ondes, dans le domaine des térahertz, c.-à-d. dans le domaine du rayonnement submillimétrique, ou dans le domaine des ondes radioélectriques, et
c) rayonnement ultrasonique.

4. Système selon la revendication 1 ou 2, dans lequel le dispositif de réception de signaux (210) comprend un récepteur pour un signal électrique, le signal électrique étant indicatif d'une capacité à mesurer et/ou d'une inductivité à mesurer, qui est caractéristique de l'état ou de l'identification du dispositif dialyseur (100) et/ou de l'installation de filtration à membrane (190).

5. Système selon la revendication 3, dans lequel le dispositif capteur (200) comprend une installation d'émission de rayonnement (240) configurée pour émettre un rayonnement prédéfini dans la zone de volume intérieur (120) du boîtier (110) du dispositif dialyseur (100), et en ce que
le dispositif de réception de signaux (210, 220, 222, 224) est configuré pour mesurer un ou plusieurs paramètres tels que l'intensité, la phase et/ou la réponse temporelle du rayonnement qui a été émis par l'installation d'émission de rayonnement (240), le rayonnement mesuré pouvant être le résultat d'une interaction caractéristique de l'état du dispositif dialyseur (200) qui s'est produite dans la zone de volume intérieur (120) du boîtier (110) entre le rayonnement et un ou plusieurs de ce qui suit :
- le dialysat ou le sang,
- une substance contenue dans le dialysat et/ou dans le sang,
- l'installation de filtration à membrane (190) et
- une substance fixée dans ou sur l'installation de filtration à membrane (190), qui provient du sang ou du dialysat,
au moins une partie du rayonnement interagissant provenant de la zone de volume intérieur (120) ayant atteint le dispositif de réception de signaux (210, 220, 222, 224),
et
l'interaction ayant été causée par un rayonnement émis par l'installation d'émission de rayonnement (240) dans la zone de volume intérieur (120) du boîtier (110).

6. Système selon la revendication 5, dans lequel est causée une interaction optique qui est sélectionnée parmi un groupe comprenant ce qui suit :
- réflexion d'un rayonnement optique émis par l'installation d'émission de rayonnement (240) au niveau d'une interface entre la paroi de boîtier (112) et le dialysat ou le sang,
- réflexion d'un rayonnement optique émis par l'installation d'émission de rayonnement (240) au niveau d'une interface entre l'installation de filtration à membrane (190) et le dialysat ou le sang,
- transmission d'un rayonnement optique émis par l'installation d'émission de rayonnement (240) avec une longueur d'ondes mesurée, qui peut être absorbé par une substance contenue dans le dialysat et/ou dans le sang et qui a parcouru sur son trajet jusqu'à l'installation de réception de rayonnement (220) un parcours de rayonnement à travers le dialysat et/ou à travers le sang,
- émission d'un rayonnement de luminescence ou de fluorescence à travers une substance contenue dans le dialysat et/ou dans le sang, une réaction de luminescence ou de fluorescence ayant été causée dans cette substance par un rayonnement optique émis par l'installation d'émission de rayonnement (240),
- réfraction d'un rayonnement optique émis par l'installation d'émission de rayonnement (240) au niveau d'une interface entre la paroi de boîtier (112) et le dialysat ou le sang, le rayonnement émis rencontrant l'interface à un angle d'incidence compris entre 0° et 180° ou 0° et 90°,
- dispersion d'un rayonnement optique émis par l'installation d'émission de rayonnement (240) au niveau d'une substance contenue dans le dialysat ou dans le sang, y compris la dispersion dynamique d'un rayonnement laser monochromatique, et
- interaction d'un rayonnement optique émis par l'installation d'émission de rayonnement (240) avec un dispositif d'identification (192) qui est monté sur le dispositif dialyseur (100) ou sur l'installation de filtration à membrane (190), qui comprend une caractéristique d'identification caractéristique de l'identification du dispositif dialyseur (100) ou de l'installation de filtration à membrane (190).

7. Système selon la revendication 5 ou 6, dans lequel l'installation d'émission de rayonnement (240) comprend au moins une ou plusieurs des caractéristiques suivantes :
- une source de lumière qui émet de la lumière dans une plage spectrale à bande étroite,
- une fibre optique qui conduit de la lumière émise à partir d'une source de lumière émettant de la lumière dans une plage spectrale à bande étroite et qui présente une section de découplage dont sort la lumière,
- une source de lumière qui émet de la lumière dans une plage spectrale à large bande,
- une fibre optique qui conduit de la lumière émise à partir d'une source de lumière émettant de la lumière dans une plage spectrale à large bande et qui présente une section de découplage dont sort la lumière.

8. Système selon la revendication 7, dans lequel l'installation d'émission de rayonnement (240) comprend un agencement à 1 dimension (231, 233) ou un agencement à 2 dimensions (236) de plusieurs zones de sortie de rayonnement (250), l'agencement (231, 233, 235), à l'état relié du dispositif capteur (200) et du dispositif dialyseur (100), étant orienté de manière transverse, oblique ou sensiblement parallèle à une direction d'écoulement du dialysat dans la région destinée au dialysat (170) ou de manière transverse, oblique ou parallèle à la direction d'écoulement du sang dans la région destinée au sang (130) ou de manière transverse, oblique ou parallèle à un axe longitudinal (102) ou suivant la circonférence du dispositif dialyseur (100).

9. Système selon la revendication 7 ou 8, dans lequel le dispositif capteur (200) comprend un dispositif de mesure de rayonnement (220) avec un dispositif d'optique de mesure qui comprend une profondeur focale et une direction focale, et avec un détecteur de lumière qui comprend une surface de détecteur photosensible, le dispositif d'optique de mesure étant configuré pour reproduire un espace de la zone de volume intérieur (120) du boîtier (110) du dispositif dialyseur (100) sur la surface de détecteur du détecteur de lumière, l'espace reproduit étant défini par la profondeur focale et la direction focale, et le dispositif d'optique de mesure étant configuré de telle sorte que sa profondeur focale et sa direction focale est réglable ou sélectionnable avant la mise en service ou pendant le fonctionnement de telle sorte que l'espace reproduit est sélectionnable dans la région de la zone de volume intérieur (120).

10. Système selon l'une des revendications précédentes, dans lequel le dispositif capteur (200) comprend un dispositif de mesure de rayonnement (220) avec au moins un détecteur de lumière et un dispositif de sélection de longueur d'ondes, le dispositif de sélection de longueur d'ondes étant configuré pour sélectionner une plage de longueur d'ondes à bande étroite comprenant une longueur d'ondes d'indication, parmi la plage de longueur d'ondes à plus large bande du rayonnement émis par l'installation d'émission de rayonnement (24), par ex. de la lumière, côté irradiation ou côté réception, de telle sorte que se produit une interaction optique avec une substance sélectionnée se trouvant en fonctionnement au niveau de ou dans le dispositif dialyseur ou dans la zone de volume intérieur (120) du boîtier (110).

11. Système selon l'une des revendications 1 à 10, dans lequel le dispositif capteur (200) est configuré pour détecter au moins un des paramètres suivants : dans la région destinée au sang (30, 130) de la zone de volume intérieur (120), mesure d'un ou de plusieurs paramètres indicatifs de la concentration d'une ou de plusieurs substances qui sont absorbés par la substance,
dans la région destinée au dialysat (70, 170) de la zone de volume intérieur (120), mesure d'un paramètre indicatif de la concentration d'une ou de plusieurs substances, dans la région destinée au sang (30, 130), mesure d'un paramètre indicatif d'une propriété physique du sang.

12. Dispositif capteur (200) configuré pour être relié de façon amovible directement à un boîtier (110) d'un dispositif dialyseur (100) et qui comprend au moins un capteur pour détecter un signal provenant de l'intérieur du boîtier, un système selon l'une des revendications 1 à 11 pouvant, dans un état relié au dispositif dialyseur (100), être formé par le dispositif capteur,
**caractérisé en ce que**
le dispositif capteur (200) est configuré comme un dispositif de clipsage (220) avec au moins un ou plusieurs bras de clipsage ou manchons de clipsage flexibles (210, 210'), qui est agencé pour entourer le boîtier (110) du dispositif dialyseur (100) suivant sa direction longitudinale dans une plage angulaire de plus de 180°, et qui comprend une deuxième zone de couplage (211, 211') qui est formée avec complémentarité de formes par rapport à une première zone de couplage (111, 111') sur le boîtier (110) du dialyseur (100),
les première et deuxième zones de couplage (111, 111', 211, 211') étant agencées pour coopérer de telle sorte que le dispositif capteur (200) peut être monté de façon amovible sur le boîtier (110) du dialyseur (100) en une position prédéterminée suivant la direction longitudinale de celui-ci.

13. Dispositif capteur (200) selon la revendication 12, comprenant au moins un émetteur de rayonnement pour l'émission d'un rayonnement sur la surface et/ou à l'intérieur du dispositif dialyseur, le dispositif capteur étant configuré réutilisable.

14. Dispositif capteur (200) selon la revendication 12 ou 13, dans lequel, pour la configuration du dispositif capteur (200) fixable au boîtier (110) de façon amovible, par ex. avec complémentarité de formes, est prévu au moins l'un des mécanismes suivants :
le dispositif capteur (200) comprend un corps de base et un dispositif de clipsage avec au moins un ou deux bras de clipsage élastiques flexibles fixés au corps de base ou bras de clipsage articulés de manière élastique flexible sur le corps de base qui sont configurés pour entourer le boîtier (110) au moins en partie ou pour établir avec celui-ci une liaison fixe amovible ou pour serrer le boîtier (110) par clipsage du fait de leur configuration élastique flexible ou de leur articulation,
le dispositif capteur (100) comprend un corps de base et au moins un bras ou une paire de bras ou manchons articulés sur le corps de base ou reliés de manière élastique flexible au corps de base, qui présentent respectivement une zone d'extrémité distale avec un premier membre qui y est pourvu d'un dispositif d'accrochage ou à crochet, les bras ou manchons étant configurés pour entourer le boîtier (110) dans une plage angulaire de plus de 180°, et un dispositif de tension pouvant être tendu élastiquement avec une ou deux sections d'extrémité opposées au niveau desquelles peut être prévu respectivement un deuxième membre d'un dispositif d'accrochage ou à crochet configuré en vue d'un accrochage amovible avec un premier membre du dispositif d'accrochage ou à crochet, un deuxième membre respectif au niveau de la section d'extrémité du dispositif de tension pouvant, dans un état tendu élastiquement du dispositif de tension, s'accrocher à un premier membre au niveau des zones d'extrémité des bras ou des manchons du dispositif capteur (200), et
le dispositif capteur (200) est configuré pour être intégré de façon amovible dans la paroi de boîtier (112) du dispositif dialyseur (100).

15. Dispositif dialyseur (100), avec un boîtier (110) renfermant une zone de volume intérieur (120) et une installation de filtration à membrane (190) agencée dans la zone de volume intérieur (120), le boîtier (110) étant perméable au moins par endroits à un signal, tel qu'un signal de rayonnement, et le dispositif dialyseur (100) étant configuré de telle sorte qu'un dispositif capteur selon l'une des revendications 12 à 14 peut lui être relié opérationnellement de façon amovible et que, dans un état relié, un système selon l'une des revendications 1 à 11 peut être formé,
**caractérisé en ce que**
le boîtier (110) du dispositif dialyseur (100) comprend sur sa face extérieure une première zone de couplage (111, 111') qui est formée avec complémentarité de formes par rapport à une deuxième zone de couplage (211, 211') sur un dispositif de clipsage (220) du dispositif capteur (200) entourant le boîtier (110) du dispositif dialyseur (100) suivant sa direction longitudinale dans une plage angulaire de plus de 180° et configuré avec au moins un ou plusieurs bras de clipsage ou manchons de clipsage flexibles (210, 210'),
les première et deuxième zones de couplage (111, 111', 211, 211') étant agencées pour coopérer de telle sorte que le dispositif capteur (200) peut être monté de façon amovible sur le boîtier (110) du dialyseur (100) en une position prédéterminée suivant la direction longitudinale de celui-ci.

16. Dispositif dialyseur (100) selon la revendication 15, avec au moins une, facultativement plusieurs ou la totalité des caractéristiques suivantes :
le boîtier (100) comprend une paroi de boîtier (112) avec au moins une zone de fenêtre (114, 116, 118) perméable à un signal ;
l'installation de filtration à membrane (190) peut être insérée enlevable dans le boîtier (100), un boîtier intérieur peut être fixé de façon amovible dans le boîtier (110) et renferme l'installation de filtration à membrane (190),
le boîtier intérieur peut être inséré enlevable dans le boîtier (100),
au moins l'installation de filtration à membrane (190) est configurée comme article à usage unique,
le boîtier intérieur et l'installation de filtration à membrane (190), ou le dispositif dialyseur (100) dans leur ensemble, sont configurés comme article à usage unique,
la zone de volume intérieur (120) comprend une région destinée au dialysat (170) pouvant être traversée en fonctionnement par un dialysat et une région destinée au sang (130) pouvant être traversée en fonctionnement par du sang, la région destinée au dialysat (170) étant séparée spatialement de la région destinée au sang (130) par l'installation de filtration à membrane (190),
l'installation de filtration à membrane (190) est configurée pour permettre en fonctionnement un échange de substances entre le sang et le dialysat, échanges provoqués par des gradients de concentration de substances dissoutes dans le sang ou
dans le dialysat,
le dispositif dialyseur est configuré comme dialyseur intelligent et / ou présente des interfaces vers un dispositif de commande externe.

17. Machine de dialyse (300) pour réaliser un traitement de dialyse sur un patient, avec un dispositif dialyseur (100) selon l'une des revendications 15 ou 16 et un dispositif capteur (200) selon l'une des revendications 12 à 14, qui sont prévus pour former un système selon l'une des revendications 1 à 11 ou pour être reliés opérationnellement de façon amovible à un système de ce type.

18. Machine de dialyse (300) selon la revendication 17, dans laquelle le dispositif dialyseur (100) comprend au moins un support de boîtier (180, 182) qui est conçu séparément ou relié fixement à la machine de dialyse et est configuré pour être relié mécaniquement au moins en fonctionnement à la machine de dialyse (300) et pour fixer le dispositif dialyseur (100) de manière amovible.

19. Machine de dialyse (300) selon la revendication 18, dans laquelle le dispositif capteur (200) est intégré dans le support de boîtier (180, 182).
